# EUROPEAN PATENT APPLICATION

(11) **EP 4 331 617 A1**
(43) Date of publication of application: **06.03.2024**
(21) Application number: 22795903.8
(22) Date of filing: 28.04.2022
(51) Int. Cl.: A61K 47/69, A61K 9/107, A61K 9/127, A61K 31/7088, A61K 31/7125, A61K 45/00, A61K 47/10, A61K 47/32, A61K 47/34, A61K 47/36, A61K 47/56, A61K 47/60, A61K 48/00, A61P 35/00, C07H 21/02, C07H 21/04, C12N 15/88

(54) **ARTIFICIAL NUCLEIC ACID AND NUCLEIC ACID DELIVERING METHOD USING SAME**

(30) Priority: 28.04.2021 JP 2021075651; 22.02.2022 JP 2022026023
(71) Applicant: Japan Science and Technology Agency, Kawaguchi-shi, Saitama 332-0012 (JP)
(72) Inventor: MIYAMOTO Noriko, Toyota-shi, Aichi 470-0392 (JP)
(74) Representative: Grünecker Patent- und Rechtsanwälte PartG mbB
(86) International application number: PCT/JP2022/019343
(87) International publication number: WO 2022/230990

(57) **Abstract**

The present invention is a nucleic acid analog in which a cationic artificial nucleic acid and a hydrophilic polymer are bonded to each other. The cationic artificial nucleic acid has a backbone structure including a constituent unit in which a base is bonded to a ring structure selected from ribose and deoxyribose, and a linking structure linking two constituent units, the linking structure, in a cationic state, has a cationic group selected from the group consisting of the following formulae (C1) to (C7), and the cationic artificial nucleic acid can associate by electrostatic interaction between a phosphate group of another nucleotide and the cationic group, wherein R₁ to R₃ represent hydrogen or an alkyl group, and may be the same or different, and Ring is a cyclic compound and may be a heterocyclic ring.

## Description

### Technical Field

The present invention relates to a nucleic acid analog, a structure for nucleic acid delivery, a method for producing the same, and a nucleic acid delivery method, and particularly relates to a structure for nucleic acid delivery, a method for producing the same, and a nucleic acid delivery method for delivering a nucleic acid to be delivered to a target site.

### Background Art

In the field of gene therapy, an oligonucleotide such as DNA or RNA is delivered to a target site such as a cell to suppress expression of a gene or recombine a gene, thereby treating a disease. In such treatment, a technology of a drug delivery system (DDS) that efficiently delivers a nucleic acid to a target site is important. Various DDS methods have been developed so far. In addition, nucleic acid analysis, diagnostic tracers, molecular machines, and molecular computers have been actively applied to the fields of nanomaterials and artificial cells.

On the other hand, for example, drug discovery targeting RAS of a cancer promoting gene associated with 30% of all cancers has been widely advanced over the past 30 years. However, since RAS protein has a narrow small molecular pocket and it is difficult to design an inhibitor, and cancer cells acquire resistance by one genetic control of RAS by antisense-oligonucleotide (AS-ODN) or siRNA, an effective therapeutic agent has not yet been developed.

Patent Literature 1 discloses a nanoparticle containing a nuclear complex encapsulated by a lipid monolayer. The complex contains one or more nucleic acid molecules electrostatically linked to one or more molecules of a cationic polymer, and the cationic polymer is covalently bonded to a first lipid residing within the lipid monolayer. Here, polyethyleneimine and the like are exemplified as the cationic polymers, and phospholipids and the like are exemplified as the first lipids, and it is described that the lipid monolayer further contains one or more non-binding lipids, and a part of the non-binding phospholipid molecules is PEGylated. Such a configuration is believed to provide nanoparticles suitable for use as an in vivo delivery agent for nucleic acids.

Patent Literature 2 describes a polyion complex containing a block copolymer containing a polyethylene glycol moiety and a polycation moiety having a thiol group side chain as a terminal as constituents, and siRNA. Here, the polycation moiety is a polypeptide having a cationic group in a side chain. Thereby, it is said that it becomes possible to provide a siRNA-containing polymeric micelle complex (polyion complex) having high monodispersibility and structural stability and excellent ability to deliver siRNA to cells.

Patent Literature 3 discloses a conjugate of a functional oligonucleotide and poly(ethylene oxide), a polyion complex of the conjugate and a cationic polymer, and a micelle thereof. The conjugate is represented by general formula (A): FuNT-L₁-L₂-PEG, and FuNT represents a residue of a functional oligonucleotide, L₁ represents an alkylene group, L₂ represents a linking group capable of being cleaved under physiological conditions, and PEG represents polyethylene glycol. The cationic polymer is selected from polylysine and the like. When a polyion complex of the conjugate and the cationic polymer is formed and incorporated into an animal cell, the conjugate cleaves and releases the functional oligonucleotide in the environment of endosome. According to this micelle, stability of the oligonucleotide in the animal cell is achieved, and specific binding to a target gene is improved.

Furthermore, the inventors have synthesized a conjugate of polyethylene glycol (PEG) and natural nucleic acid (DNA) for the purpose of developing application to DDS (Non Patent Literature 1).

### Citation List

### Patent Literature

Patent Literature 1: JP 2011-503070 W (abstract, claims 1 to 6, Fig. 1, paragraph 0060, and the like)
Patent Literature 2: WO 08/062909 A (abstract, claim 1, and the like)
Patent Literature 3: WO 05/100447 A (abstract, claim 1, claim 8, and the like)
Non Patent Literature 1: "Development of immune evasion type PMO/DNA nanostructure and application of DDS", Project Result Report in FY 2017
(https://kaken.nii.ac.jp/ja/report/KAKENHI-PROJECT-17H07219/17H072192017j isseki/)

### Summary of Invention

### Technical Problem

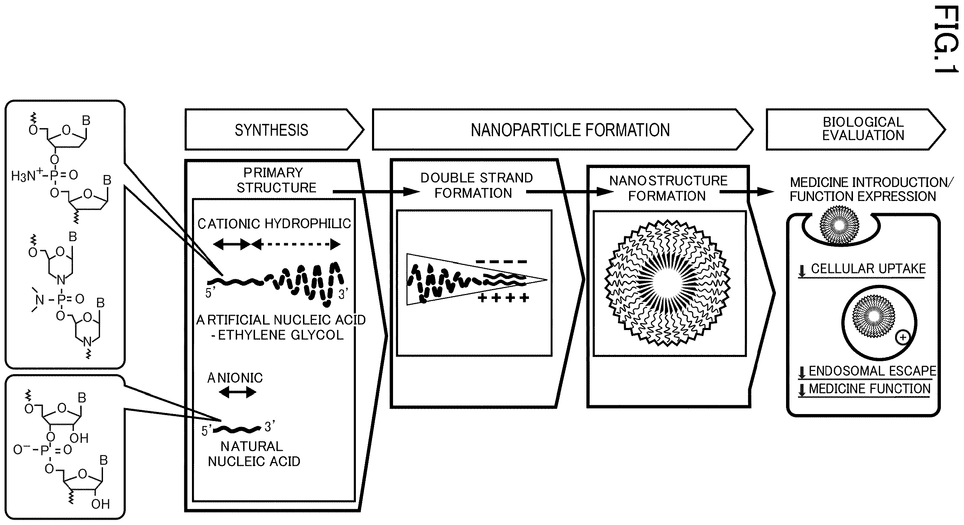

The complex of Patent Literature 1 uses a polymer compound such as polyethyleneimine as a cationic polymer, but has weak interaction with a nucleic acid to be delivered. In addition, in this literature, the nucleic acid (DNA) to be delivered and the cationic polymer are electrostatically bound to each other, but the DNA to be delivered is double-stranded (see Fig. 1 of this literature), and it is not possible to bind to the nucleic acid in the state of single-stranded DNA and deliver the nucleic acid to the target site.

In addition, the complex of this literature has a structure in which a cationic polymer is covalently bonded to an amphiphilic lipid, and a nanostructure formed by the complex (preformed complex) contains a non-binding lipid such as PEG-PE, thereby imparting in vivo stability (see paragraph 0060, Fig. 1). That is, in this literature, since the nanostructure itself formed by the complex itself is not PEGylated, the nanostructure does not have sufficient in vivo stability such as blood retention, and the PEG of the additionally encapsulated non-binding lipid imparts in vivo stability. The non-bound lipid is a lipid not bound to the cationic polymer, and this literature does not disclose a molecule in which the cationic polymer and PEG are bonded.

In the block copolymer of Patent Literature 2, since the polycation moiety is a polypeptide, the interaction with the siRNA to be delivered is weak for the same reason as in Patent Literature 1, and the block copolymer cannot bind to the siRNA in a single-stranded state.

The conjugate of Patent Literature 3 is one in which a functional oligonucleotide (FuNT) and PEG are bonded, but this functional oligonucleotide is not cationic but anionic nucleotide. In addition, the conjugate of this literature is for releasing a functional oligonucleotide by cleavage of a linking group, and itself is a delivery target and not a carrier for carrying nucleic acids.

As described above, conventionally, a carrier capable of binding to a single-stranded nucleic acid to be delivered and delivering the nucleic acid to a target site and having a structure formed by association or the like with high in vivo stability by itself has not been known.

The DNA-PEG conjugate of Non Patent Literature 1 forms a complementary double strand by hydrogen bonding between bases and π-π stacking interaction with a nucleic acid to be delivered. Therefore, it is suitable for delivery of a nucleic acid having a sequence complementary to the DNA of the conjugate. On the other hand, a DNA-PEG conjugate suitable for delivery of nucleic acids of non-complementary sequence has not been known so far.

An object of the present invention is to provide a nucleic acid analog that can be associated with not only a complementary sequence but also a single-stranded nucleic acid of non-complementary sequence and delivered to a delivery site. Also, another object of the present invention is to provide a structure for nucleic acid delivery formed by such a nucleic acid analog, a method for producing a nucleic acid analog or a structure for nucleic acid delivery, and a nucleic acid delivery method using a nucleic acid analog.

### Solution to Problem

The present inventors have intensively studied to solve the above problems. As a result, the present inventors have paid attention to the fact that in vivo, a higher-order structure is precisely formed by electrostatic interaction between genomic DNA and histone proteins and stored in the nucleus. Then, the present inventors have found that by introducing a cationic group into a portion of a linking structure of nucleic acid (or nucleic acid analog) of a conjugate, the nucleic acid associates with a nucleic acid to be delivered through electrostatic interaction, thereby completing the present invention.

[1] A nucleic acid analog containing a cationic artificial nucleic acid and a hydrophilic polymer bonded to the cationic artificial nucleic acid,
   the cationic artificial nucleic acid having a backbone structure including a constituent unit in which a base is bonded to a ring structure selected from ribose and deoxyribose, and a linking structure linking the two constituent units,
   the linking structure, in a cationic state, having a cationic group selected from the group consisting of the following formulae (C1) to (C7), and the cationic artificial nucleic acid being capable of associating by electrostatic interaction between a phosphate group of another nucleotide and the cationic group, 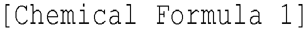 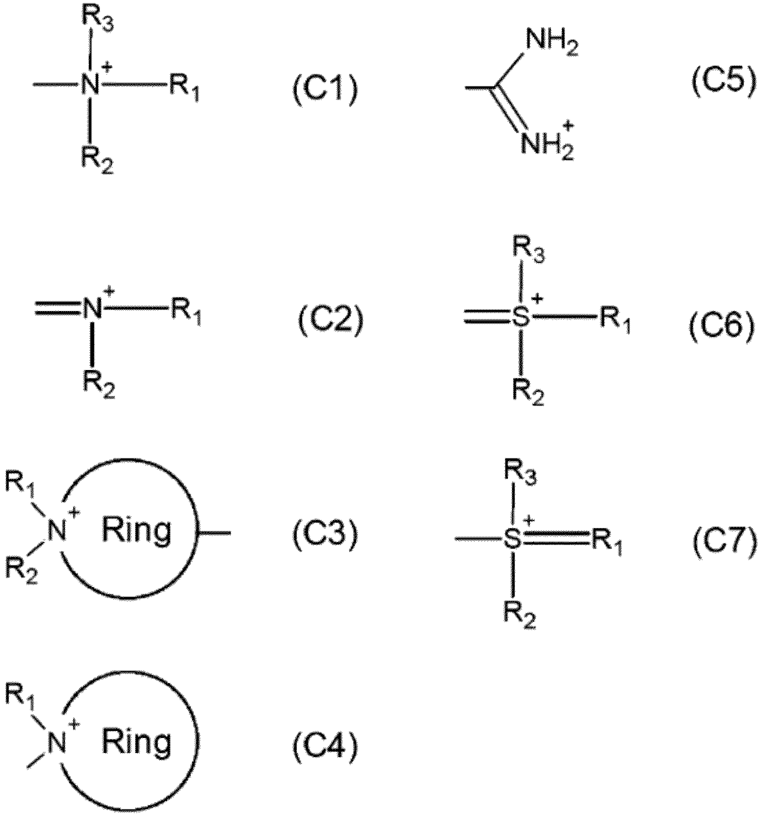
   wherein R₁ to R₃ represent hydrogen or an alkyl group having 1 to 10 carbon atoms, R₁ to R₃ may be the same or different, and Ring is a cyclic compound composed of 4 to 8 carbon atoms, and may be a heterocyclic ring in which one or more of the carbon atoms are substituted with a heteroatom selected from nitrogen, oxygen, and sulfur.
[2] The nucleic acid analog according to [1], in which the cationic group has a pKa in the range of 6 to 9.
[3] The nucleic acid analog according to [1], in which the linking structure, in a cationic state, has at least a structure selected from the following formulae (L1) to (L4): 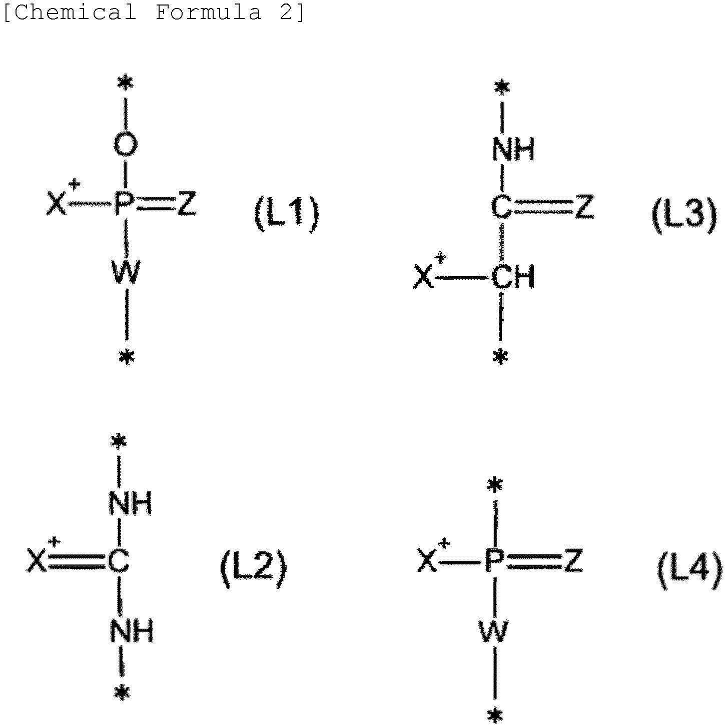 wherein X⁺ is a functional group containing the cationic group, Z represents O or S, W represents -O- or -NR₄-, and R₄ represents hydrogen or an alkyl group having 1 to 10 carbon atoms, and * means a bond with the constituent unit adjacent to each other.
[4] The nucleic acid analog according to [1], in which the cationic artificial nucleic acid has a nucleotide backbone represented by the following formula (N1) : 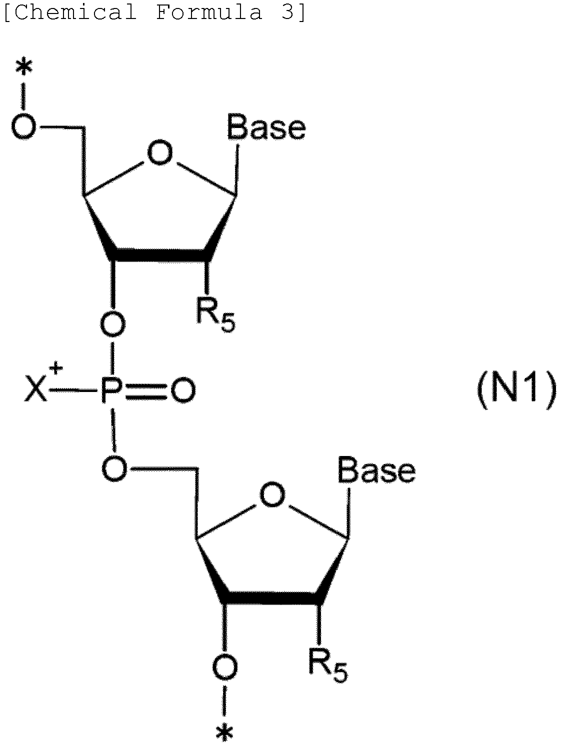 wherein X⁺ is a functional group containing the cationic group, Base represents a base, and R₅ represents H or OH, * means a bond with phosphoric acid of an adjacent nucleotide backbone, and at least one of * is bonded to the hydrophilic polymer at the 5' end or 3' end, and when not bonded to the hydrophilic polymer, * is hydrogen.
[5] The nucleic acid analog according to [4], in which the X⁺, in a cationic state, is an ammonium cation represented by the following formula (F1): 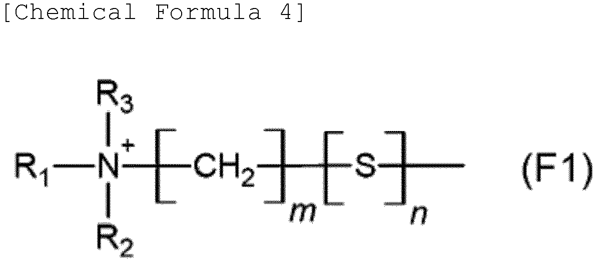 wherein R₁ to R₃ represent hydrogen or an alkyl group having 1 to 10 carbon atoms, and may be the same or different from each other, m represents an integer of 0 to 10, and n represents an integer of 0 or 1.
[6] The nucleic acid analog according to [1], in which the hydrophilic polymer is selected from polyethylene glycol, polyvinyl alcohol, polyglutamic acid, polyvinylpyrrolidone, polyacrylamide, polyethyleneimine, polyalkyl acrylate, polyoxazoline, polyacrylamide, poly(carboxybetaine methacrylate), poly(sulfobetaine methacrylate), poly(2-methacryloyloxyethyl phosphocholine), hyaluronic acid, chitosan, dextran, and derivatives thereof.
[7] The nucleic acid analog according to [7], in which the hydrophilic polymer has a polyethylene glycol backbone represented by the following formula (A1): 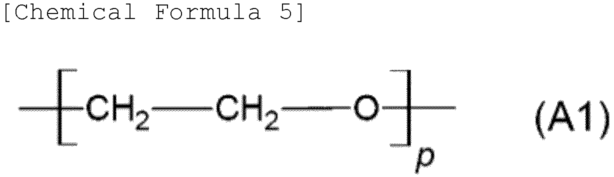 wherein p represents an integer of 1 to 20.
[8] The nucleic acid analog according to [1], which is a carrier for delivering a nucleic acid to be delivered to a target site.
[9] A structure for nucleic acid delivery including the nucleic acid analog according to [8] and a nucleic acid to be delivered, for delivering the nucleic acid to a target site, the structure for nucleic acid delivery having an association structure in which the cationic artificial nucleic acid and the nucleic acid are associated by electrostatic interaction.
[10] The structure for nucleic acid delivery according to [9], in which the structure for nucleic acid delivery is a nanoscale structure in which a plurality of the structures for nucleic acid delivery are associated with each other.
[11] The structure for nucleic acid delivery according to [10], which is a vesicle or a micelle in which the association structure is located on an inner side and the hydrophilic polymer is located on an outer side.
[12] The structure for nucleic acid delivery according to [11], which is modified with a ligand.
[13] The structure for nucleic acid delivery according to [11], further having a medicine.
[14] The structure for nucleic acid delivery according to [13], in which the structure for nucleic acid delivery is a spherical structure having a hollow portion in a central portion, and
   a medicine is enclosed in the hollow portion.
[15] A method for producing the nucleic acid analog according to [1], including: a cationic nucleic acid synthesis step of synthesizing the cationic artificial nucleic acid; and a binding step of binding the cationic artificial nucleic acid and the hydrophilic polymer, and the cationic nucleic acid synthesis step including: a step of introducing a thiophosphate ester into the linking structure; and a step of reacting the thiophosphate ester with a bromo compound.
[16] A method for producing the structure for nucleic acid delivery according to [9], including:
   an association step of associating the nucleic acid analog and the nucleic acid by electrostatic interaction under a pH condition lower than pKa of the cationic group of the nucleic acid analog and higher than pKa of phosphoric acid of the nucleic acid to form an association nucleic acid structure.
[17] A method for producing the structure for nucleic acid delivery according to [16], further including: an associate forming step of associating a plurality of the structures for nucleic acid delivery to form, in an aqueous solvent, a vesicle or a micelle in which the association structure is located on an inner side and the hydrophilic polymer is located on an outer side.
[18] A nucleic acid delivery method for delivering the nucleic acid to a target site using the structure for nucleic acid delivery according to [9], the nucleic acid delivery method including:
   an administration step of administering the structure for nucleic acid delivery to incorporate the structure for nucleic acid delivery into the target site; and
   a releasing step of releasing the nucleic acid in the target site.

[A] The nucleic acid analog according to [1], in which the cationic artificial nucleic acid has a morpholino backbone represented by the following formula (M1) : 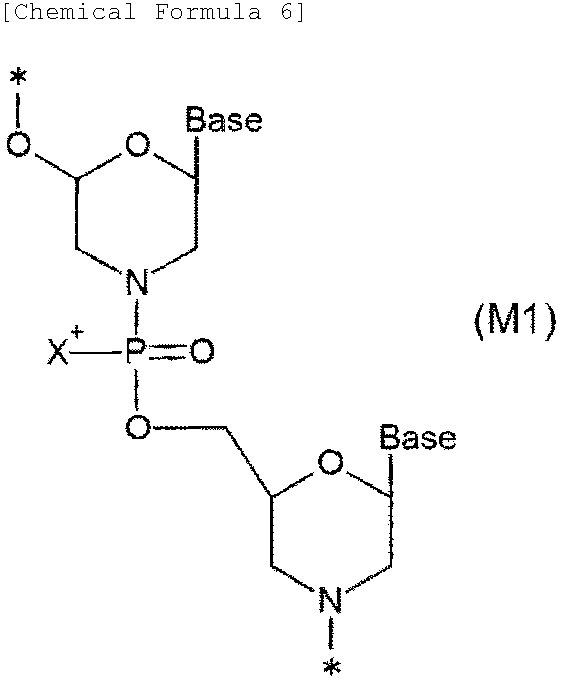 wherein Base represents a base, * means a bond with phosphorus of an adjacent morpholino backbone, and at least one of * is bonded to the hydrophilic polymer at the 5' end or 3' end, and when not bonded to the hydrophilic polymer, * is hydrogen.

### Advantageous Effects of Invention

According to the present invention, it is possible to provide a nucleic acid analog that can be associated with not only a complementary sequence but also a single-stranded nucleic acid of non-complementary sequence by electrostatic interaction and delivered to a delivery site. Furthermore, according to the present invention, it is possible to provide a structure for nucleic acid delivery formed by such a nucleic acid analog, a method for producing a nucleic acid analog or a structure for nucleic acid delivery, and a nucleic acid delivery method using a nucleic acid analog.

### Brief Description of Drawings

Fig. 1 is a diagram illustrating an outline of a nucleic acid analog and a structure for nucleic acid delivery of the present invention.
Fig. 2 is a diagram illustrating an example of a synthesis scheme of a cationic artificial nucleic acid.
Fig. 3 is a diagram illustrating an example of a synthesis scheme of a nucleic acid analog.
Fig. 4 is a diagram illustrating experiments and results of evaluating formation of structural grounds in Examples. PAGE images (A, B) The left figure is a fluorescence image in which Cy3 of SEQ ID 1' of AS chain was detected. Here, in SEQ ID 1', SEQ ID 1 and SEQ ID 2 are mixed at 10: 1. The right figure is an image after RNA staining. Electrophoretic results of (A) annealing mixture of AN4, (B) annealing mixture of AN5. (C) View of AN4 observed by TEM. Scale bar: 100 nm. (D) Result of observation of AN5 by AFM. (E, F) Particle size evaluation by DLS measurement.
Fig. 5 is a diagram illustrating experiments and results of pH response evaluation and serum resistance evaluation of structures in Examples. Results for CD (A, B). Chirality evaluation of nucleic acids at pH 7 and pH 5 of (A) AN4 and (B) AN5. Results of DLS measurement (C, D). Particle size evaluation at pH 7 and pH 5 of (C) AN4 and (D). Results of DLS measurements upon incubation with serum (E).
Fig. 6 is a diagram illustrating experiments and results of cell evaluation of nucleic acid delivery by structures in Examples. (A) Luciferase inhibitory activity evaluation of DLD-1. (B, C) Evaluation of microRNA delivery to DLD-1. Cell viability (B), expression level of K-RAS protein (C).
Fig. 7 is a view illustrating results of confocal laser microscope observation in Examples. (A) NT, (B) Naked, (C) AN3, (D) AN4, (E) AN5, (F) AN7, (G) AN8, (H) AN9. (I) Observation images of AN4 at 120-fold magnification. Scale bar: 50 µm.
Fig. 8 is a view illustrating experiments and results of evaluating structure formation of a morpholino nucleic acid (PMO)-PEG conjugate in Examples.
Fig. 9 is a view illustrating experiments and results of non-complementary strand annealing in Examples. (A) Results of electrophoresis of AN25 to 27, (B) confocal microscope observation of AN26 structure, (C) inhibition evaluation of renilla luciferase (RL) activity of AN26, and (D) evaluation of MCF-7 cellular uptake of AN26.
Fig. 10 is a diagram illustrating results of HPLC in Examples.
Fig. 11 is a diagram illustrating results of luciferase inhibition evaluation of MCF-7 in Examples.
Fig. 12 is a diagram illustrating results of cell viability evaluation of MCF-7 in Examples.
Fig. 13 is a diagram illustrating results of expression levels of K-RAS protein in MCF-7 in Examples.
Fig. 14 is a view illustrating in vivo evaluation results using a DLD-1 tumor-bearing model mouse in Examples, in which (A) illustrates an antitumor effect, and (B) illustrates kinetic evaluation.
Fig. 15 is (a) a diagram illustrating an outline of nanostructure after ligand introduction in Examples, (A) sequence design, (B) sequence synthesis, and is (b) a diagram illustrating a behavior on HPLC of artificial nucleic acids after ligand introduction in Examples. (c) shows (C) physiological activity when nanostructure AN30 in Examples is formed.
Fig. 16 illustrates (D) cellular uptake of nanostructure AN4 and nanostructure AN30 after ligand introduction in Examples.
Fig. 17 illustrates (B) cellular uptake of nanostructure AN8 and nanostructure AN31 after ligand introduction in Examples.

### Description of Embodiments

### 1. Nucleic acid analogue

Hereinafter, the nucleic acid analog of the present invention will be described. The nucleic acid analog of the present invention includes a cationic artificial nucleic acid and a hydrophilic polymer bound to the cationic artificial nucleic acid. The nucleic acid analog is preferably used as a carrier for delivering a nucleic acid that is to be delivered (hereinafter, may be referred to as a "nucleic acid to be delivered") to a target site. Hereinafter, the nucleic acid analog will be described in detail.

### (1) Cationic artificial nucleic acid

The cationic artificial nucleic acid has a backbone structure including a constituent unit in which a base is bonded to a ring structure selected from ribose and deoxyribose, and a linking structure linking two constituent units. The linking structure, in a cationic state, has a cationic group selected from the group consisting of the following formulae (C1) to (C7): 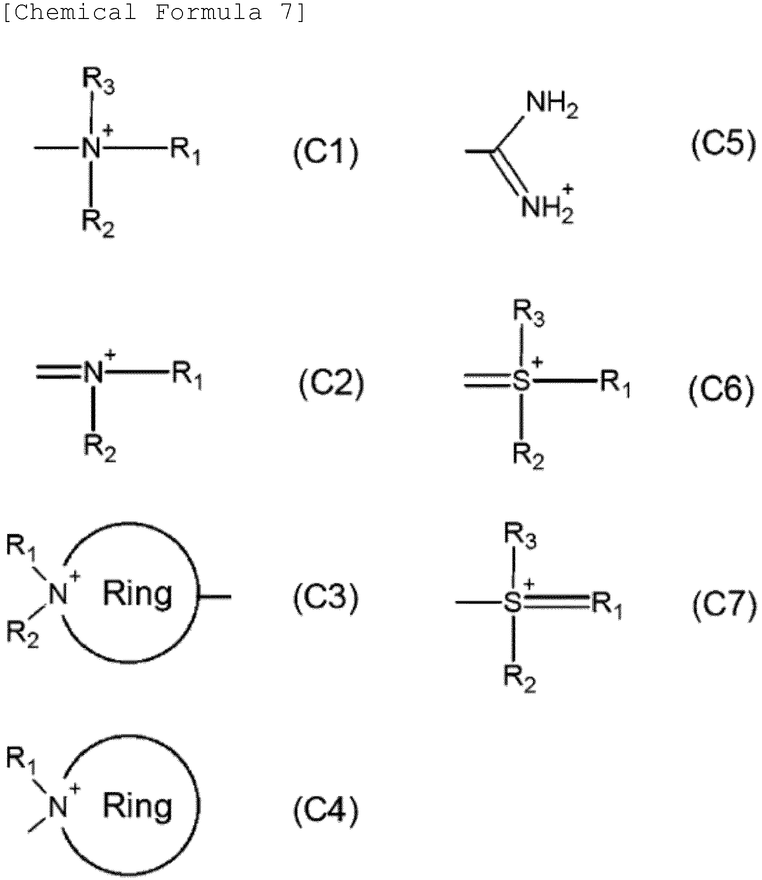 wherein R₁ to R₃ represent hydrogen or an alkyl group having 1 to 10 carbon atoms, R₁ to R₃ may be the same or different, and Ring is a cyclic compound composed of 4 to 8 carbon atoms, and may be a heterocyclic ring in which one or more of the carbon atoms are substituted with a heteroatom selected from nitrogen, oxygen, and sulfur.

The cationic artificial nucleic acid can associate by electrostatic interaction between a phosphate group of another nucleotide and the cationic group. Here, the "another nucleotide" means a nucleotide such as DNA or RNA or an analog thereof, and when a nucleic acid analog is used as a carrier, it means a nucleotide constituting a nucleic acid to be delivered or an analog thereof.

The pKa of the cationic group is higher than the pKa of the phosphate group of another nucleotide from the viewpoint of electrostatic interaction with the phosphate group of another nucleotide. Under a pH condition lower than the pKa of the cationic group of the nucleic acid analog and higher than the pKa of the phosphate group of another nucleotide, the cationic group is positively charged and the phosphate group is negatively charged, so that these groups are electrostatically bonded. Here, in general, since the pKa of the phosphate group of the nucleotide is less than 1, the pKa of the cationic group is 1 or more, preferably 3 or more, and more preferably 6.0 or more. The upper limit of the pKa of the cationic group is not particularly limited, but is 12 or less, preferably 11 or less, and more preferably 9 or less. The pKa of the cationic group is preferably within the range of 6 to 9 from the viewpoint of the strength of electrostatic interaction with the phosphate group of another nucleotide, the structure of the cationic artificial nucleic acid, and the like.

Examples of the base include adenine, guanine, cytosine, thymine, uracil, N-methyladenine, N-benzoyladenine, 2-methylthioadenine, 2-aminoadenine, 7-methylguanine, N-isobutyrylguanine, 5-fluorocytosine, 5-bromocytosine, 5-methylcytosine, 4-N-methylcytosine, 4-N,N-dimethylcytosine, 5-fluorouracil, 5-bromouracil, 5-chlorouracil, 5,6-dihydrouracil, and the like.

The linking structure, in a cationic state, is preferably one having at least a structure selected from the following formulae (L1) to (L4): 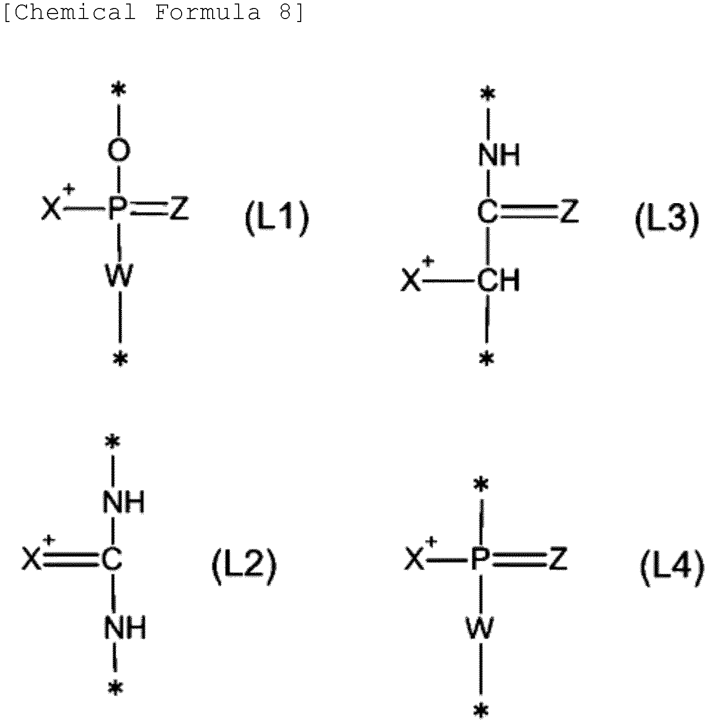 wherein X⁺ is a functional group containing the cationic group of the above formulae (C1) to (C7), Z represents O or S, W represents -O- or -NR₄-, and R₄ represents hydrogen or an alkyl group having 1 to 10 carbon atoms, and " means a bond with the adjacent constituent unit described above.

Here, the phrase "in a cationic state" means a case where the cationic group is assumed to be positively charged, and there is a case where the cationic group is not positively charged depending on environmental conditions (pH, type, and the like of the solvent) in which the nucleic acid analog is placed. When the cationic group is ammonium taking the above formula (C1) as an example, examples of the state of not being positively charged include states of a primary amine (primary ammonium when cationized), a secondary amine (secondary ammonium when cationized), a tertiary amine (tertiary ammonium when cationized), and the like.

Examples of the cationic artificial nucleic acid include those having a nucleotide backbone having, as a constituent unit, a nucleotide in which a base is bonded to ribose or deoxyribose. For reference, the cationic artificial nucleic acid also includes those having a morpholino backbone having, as a constituent unit, a structure in which a base is bonded to morpholine.

Examples of those having a nucleotide backbone include a structure represented by the following formula (N1) : 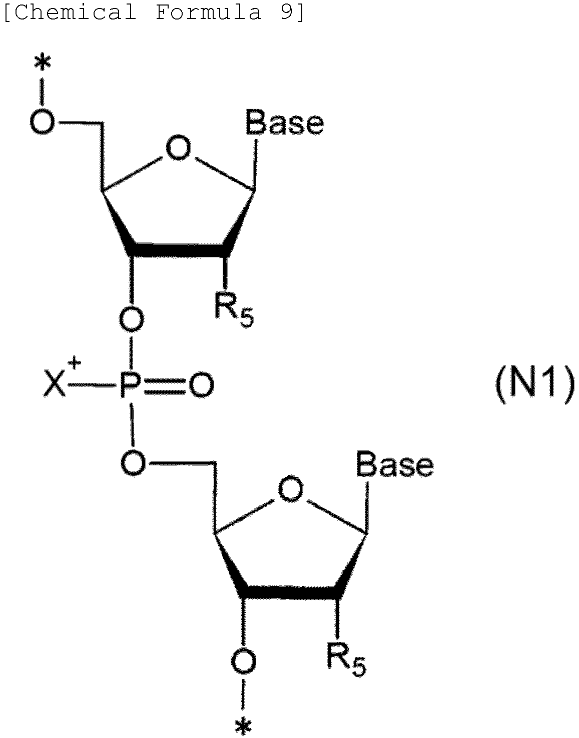 wherein Base represents a base, and R₅ represents H or OH, * means a bond with phosphoric acid of an adjacent nucleotide backbone, and at least one of * is bonded to the hydrophilic polymer at the 5' end or 3' end, and when not bonded to the hydrophilic polymer, * is hydrogen.

Examples of the structure having a nucleotide backbone include the following.

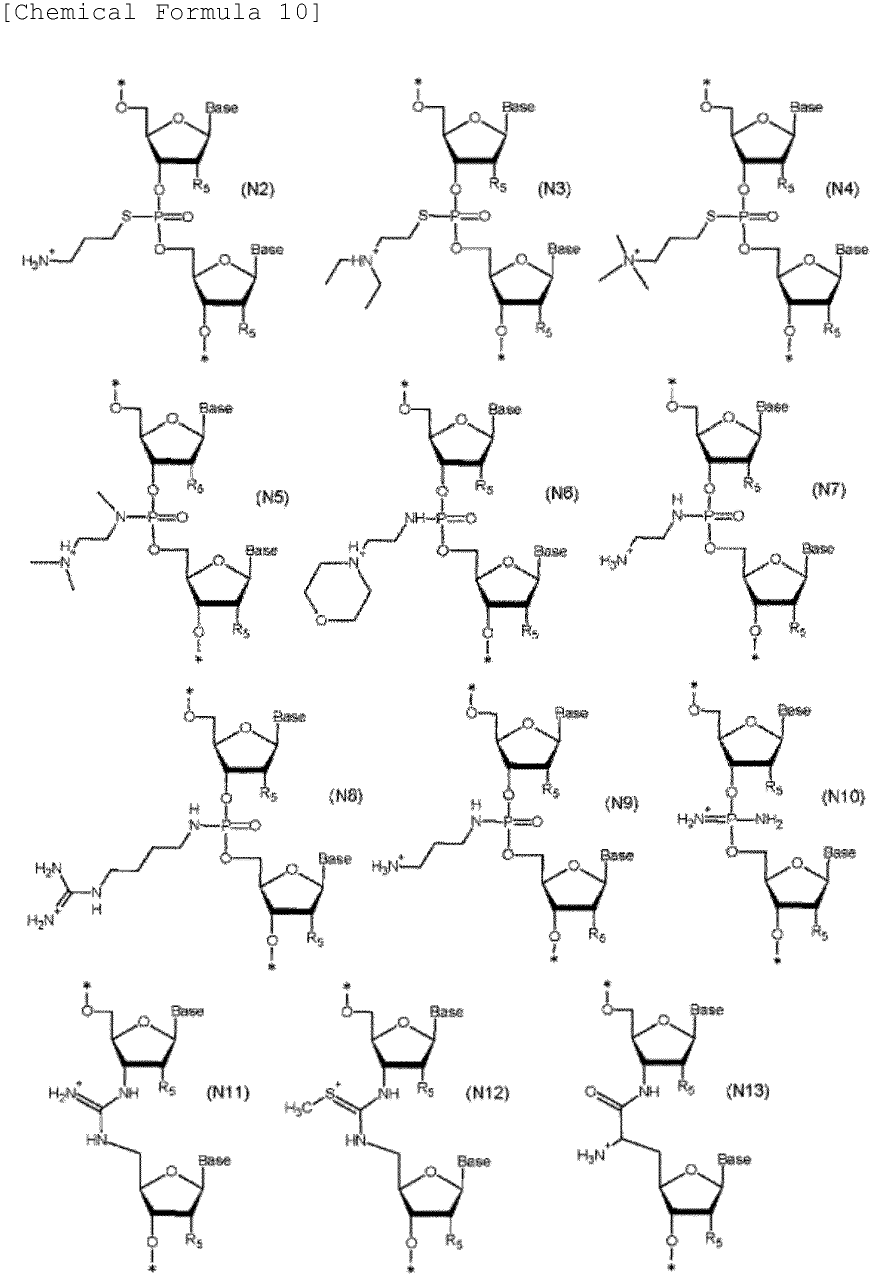

In the above case, X⁺, in a cationic state, is preferably an ammonium cation represented by the following formula (F1): 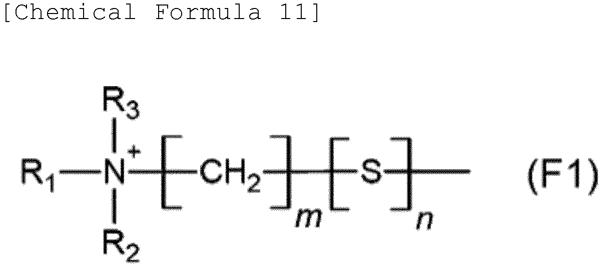 wherein R₁ to R₃ represent hydrogen or an alkyl group having 1 to 10 carbon atoms, and may be the same or different from each other, m represents an integer of 0 to 10, and n represents an integer of 0 or 1.

Here, the strength of electrostatic interaction with the phosphate group of another nucleotide is primary amine (R₁ to R₃ are all hydrogen) < secondary amine (two of R₁ to R₃ are hydrogen, and the other is an alkyl group) < tertiary amine (one of R₁ to R₃ is hydrogen, and the others are alkyl groups) < quaternary ammonium (R₁ to R₃ are all alkyl groups). In addition, as shown in Examples described later, a structure having quaternary ammonium as a cationic group has a property of being likely to collapse as the pH of the environment decreases, as compared with one having tertiary amine and the like. This means that if a structure having quaternary ammonium as a cationic group is transferred to cytoplasm via endosome, the structure is likely to collapse in response to a weakly acidic endosomal pH and release a nucleic acid to be delivered. Therefore, among these, the cationic group of X⁺ is particularly preferably quaternary ammonium. The pKa of the cationic group is about 6.1 to 7.9 in the case of primary amine, 6.9 to 7.0 in the case of secondary amine, 8.0 to 8.6 in the case of tertiary amine, and about 8.0 to 9.0 in the case of quaternary ammonium, depending on the structure.

On the other hand, examples of the structure having a morpholino backbone can include a structure represented by the following formula (M1): 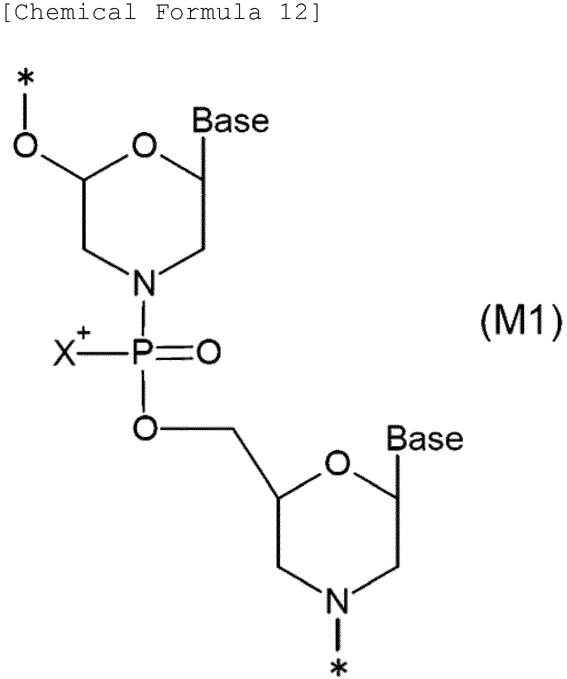 wherein Base represents a base, * means a bond with phosphorus of an adjacent morpholino backbone, and at least one of * is bonded to the hydrophilic polymer at the 5' end or 3' end, and when not bonded to the hydrophilic polymer, * is hydrogen.

Examples of the structure having a morpholino backbone include the following.

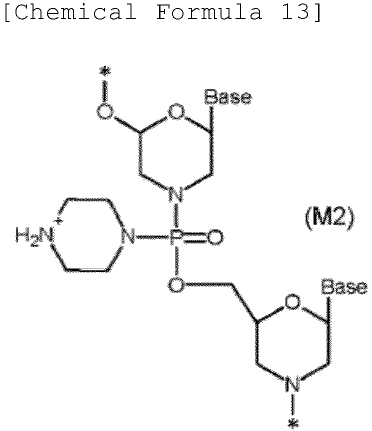

The number of constituent units in which a ring structure and a base constituting the cationic artificial nucleic acid are bonded to each other (degree of polymerization) can be appropriately set according to conditions such as the type of the ring structure and the cationic group, and the type and length (number of bases) of the nucleic acid to be delivered. In general, the degree of polymerization of the cationic artificial nucleic acid is about 5 to 100, preferably about 10 to 50. When the degree of polymerization (number of bases) of the nucleic acid to be delivered is as short as about 20 bases, it is preferable that the degree of polymerization of the cationic artificial nucleic acid as a carrier is also about the same as that of the nucleic acid to be delivered. On the other hand, when a relatively long nucleic acid such as mRNA is assumed to be delivered, the ratio of the negative charge to the length of the nucleic acid is small, and the influence of a portion having a negative charge on the entire nucleic acid is small. Therefore, the degree of polymerization of the cationic artificial nucleic acid may be different from the degree of polymerization of the nucleic acid to be delivered as long as the association between the cationic artificial nucleic acid and the nucleic acid to be delivered is not hindered. For example, when the nucleic acid to be delivered is a long nucleic acid such as mRNA, the degree of polymerization of the cationic artificial nucleic acid may be smaller than the degree of polymerization of the nucleic acid to be delivered.

In the cationic artificial nucleic acid, the cationic group is introduced into a part or whole of a linking structure that bonds constituent units, but from the viewpoint of structure formation of a vesicle and a micelle to be described later and the like, the ratio of the linking structure into which the cationic group is introduced is preferably 50% or more, more preferably 80% or more, and particularly preferably 100% (whole linking structure) with respect to the total number of linking structures. When the ratio of the number of linking structures into which a cationic group is introduced to the total number of linking structures is low, in the association structure formed between the cationic artificial nucleic acid and the nucleic acid to be delivered, the negative charge of the nucleic acid to be delivered becomes dominant, and a structure is hardly formed. Alternatively, when a cationic group is sequentially introduced into one of the 3' and 5' ends, or when a cationic group is introduced into an intermittent linking structure such as an alternate linking structure, it is conceivable to form a structure, but it is considered that the pH response of these structures changes depending on the introduction ratio of cations, and the structure is unstable. Therefore, the ratio of the linking structure into which the cationic group is introduced is preferably high.

### (2) Hydrophilic polymer

As the hydrophilic polymer, various polymers can be used depending on the use of the nucleic acid analog and the like. In particular, when a nucleic acid analog is used as a carrier for delivering a nucleic acid to a target site, the hydrophilic polymer preferably has biocompatibility. In addition, since the nucleic acid analog is cationic, the hydrophilic polymer is preferably a neutral polymer which hardly electrically interacts (suction, repulsion) with the nucleic acid analog.

Such a hydrophilic polymer is preferably selected from polyethylene glycol, polyvinyl alcohol, polyglutamic acid, polyvinylpyrrolidone, polyacrylamide, polyethyleneimine, polyalkyl acrylate, polyoxazoline, polyacrylamide, poly(carboxybetaine methacrylate), poly(sulfobetaine methacrylate), poly(2-methacryloyloxyethylphosphocholine), hyaluronic acid, chitosan, dextran, and derivatives thereof.

Examples of the hydrophilic polymer include the following.

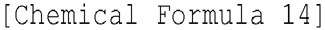

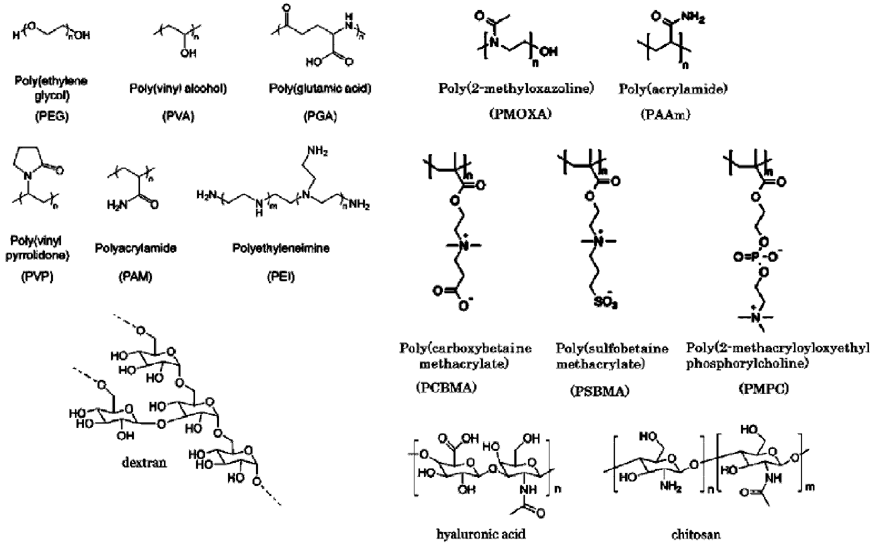

The type of monomer constituting the hydrophilic polymer and the number of monomer units (degree of polymerization) can be appropriately set according to conditions such as the surrounding environment of the target site to be delivered. In general, the degree of polymerization of the hydrophilic polymer is about 3 to 100, preferably about 10 to 50. When the degree of polymerization of the hydrophilic polymer is low, it is difficult to form a structure to be described later, and the hydrophilic polymer is likely to be removed as a foreign substance in a living body, so that decomposition stability and blood retention tend to be low. Conversely, when the degree of polymerization of the hydrophilic polymer is too high, the size of the structure is too large, and the delivery efficiency of the nucleic acid to be delivered to the target site tends to be low.

In particular, when a nucleic acid analog is used as a carrier of a drug delivery system (DDS), polyethylene glycol is particularly preferable as the hydrophilic polymer from the viewpoint of blood retention and the like. As the polyethylene glycol, a 10 to 100 K polyethylene glycol may be used, but it is preferable to have a polyethylene glycol backbone represented by the following formula (A1): 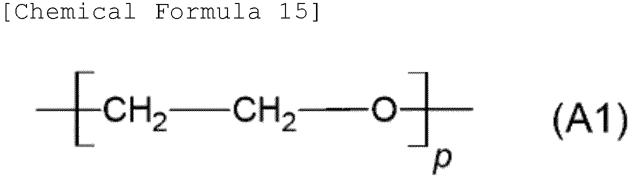 wherein p represents an integer of 1 to 20.

A1 can also be used as a polymer having a nucleobase or a phosphate ester derivative (-PO(OH)-, - PS(OH)-, PO(SH)-) as a monomer unit. The number of repetitions q of ethylene glycol of the monomer unit A1 is 1 to 20, preferably 3 to 10, the structure of the monomer may be the same or different, and the degree of polymerization of the monomer unit is 1 to 10, preferably 2 to 10, and further preferably 2 to 8. Examples of such a phosphate ester derivative monomer unit include an ethylene glycol phosphate ester derivative unit, a diethylene glycol phosphate ester derivative unit, a triethylene glycol phosphate ester derivative unit, a hexaethylene glycol phosphate ester derivative unit, and the like. These derivative units can also be used as monomers of a polymer. The degree of polymerization of the phosphate ester derivative unit is 1 to 10, preferably 2 to 10, and further preferably 2 to 8 when the number of bases of the artificial nucleic acid is 10 to 30, depending on the size of the artificial nucleic acid. It is most preferable that the monomer unit is a triethylene glycol phosphate ester derivative or a hexaethylene glycol phosphate ester derivative. The type and degree of polymerization of the monomer of the hydrophilic polymer described above, particularly the degree of polymerization of the ethylene glycol phosphate derivative monomer, can be selected from the viewpoints of easiness of formation of a structure to be described later, uneasiness of recognition as a foreign substance in a living body, lowering of decomposition stability and blood retention, and the like.

The bond between the cationic artificial nucleic acid and the hydrophilic polymer may be bonded in various bonding modes, or may be bonded via a linker. Examples of such a bond include an ester bond (-C(=O)-O-), an ether bond (-O-), a disulfide bond (-S-S-), phosphoramide (-P(OH)-NH-), phosphoric acid ester (-OPO-O-), and the like. As the linker, these may be used alone or in combination, and an ester bond, an ether bond, a disulfide bond or the like of an alkyl chain may be interposed therebetween. When the cationic artificial nucleic acid is a nucleotide backbone, it is preferable that these linkers are bonded to a hydroxyl group at the 5' end or 3' end or hydroxyl groups at both of them of the cationic artificial nucleic acid, and are bonded to the cationic artificial nucleic acid via a hydrophilic polymer. When the cationic artificial nucleic acid and the hydrophilic polymer are bonded to each other by a linker, the linker is different in structure from the hydrophilic polymer, so that it can be expected that the charge is regulated. In addition, in the case of interposing a phosphoramidite, it is preferable because it behaves as a negative charge similarly to a polymer made of polyethylene glycol. Further, it is also advantageous in that the induction of PEG antibody is suppressed. Furthermore, in the case of interposing a phosphoramidite, it is industrially advantageous in that the nucleic acid sequence to the hydrophilic polymer portion can be consistently synthesized by automatic nucleic acid synthesis.

### 2. Carrier for delivery of nucleic acid target nucleic acid, structure for nucleic acid delivery

The nucleic acid analog of the present invention can be particularly preferably used as a carrier for delivering a nucleic acid to be delivered to a target site. Fig. 1 is a schematic diagram illustrating a case where a nucleic acid analog is used as a carrier. As shown in this figure, the nucleic acid analog has a cationic group and a hydrophilic polymer as primary structures, and the nucleic acid to be delivered such as a natural nucleic acid has anionic properties (see "SYNTHESIS" in the figure). An association structure in which the anionic nucleic acid to be delivered and the cationic group of the nucleic acid analog are associated with each other by electrostatic interaction is formed to form a complex (ion complex) composed of the nucleic acid analog as a carrier and the nucleic acid to be delivered (see "DOUBLE STRAND FORMATION" in "NANOPARTICLE FORMATION" in the figure).

Here, when there is complementarity between the nucleic acid to be delivered and the cationic group, a double strand is formed by hydrogen bonding between bases, but in the present invention, even when the complementarity is low, it is excellent in that the cationic group and the phosphate group of the nucleic acid to be delivered can associate with each other by electrostatic interaction. That is, not only a nucleic acid to be delivered having complete complementarity (100% match) but also, for example, a nucleic acid to be delivered having complementarity of 80% or 90% forms an association structure by the electrostatic interaction to form a complex. Therefore, the nucleic acid to be delivered which is a non-complementary strand can be delivered to the target site. The degree of complementarity between the cationic artificial nucleic acid and the nucleic acid to be delivered is preferably 50% or more, more preferably 80% or more, and particularly preferably 100%, depending on the type of cationic group and the like.

In an aqueous environment, such as in blood, segments of the hydrophilic polymer of the complex associate each other to form a nanoscale structure for nucleic acid delivery (hereinafter, it may be simply referred to as a "structure"). As the nanoscale structure, there is a structure such as a micelle or a vesicle in which a segment of an association structure is located on an inner side and a segment of a hydrophilic polymer is located on an outer side (see "NANOSTRUCTURE FORMATION" in the figure). These micelles and vesicles form a spherical structure having a hollow portion in a central portion, and a medicine such as a low molecular weight compound can be enclosed in the hollow portion. This makes it possible to deliver not only the nucleic acid to be targeted but also a small molecule medicine. The diameter of the hollow portion is about 50 to 500 nm.

The micelle has a structure in which the segment of the hydrophilic polymer is located in the outermost shell of the spherical structure, and the segment of the association structure is located at a position facing the hollow portion at the center of the spherical structure. On the other hand, the vesicle has a double membrane structure in which two complexes are associated with each other via the segment of the association structure, and one segment of the hydrophilic polymer of the double membrane is located in the outermost shell of the spherical structure, and the other segment of the hydrophilic polymer is located at a position facing the hollow portion at the center of the spherical structure. For this reason, it is preferable to enclose a hydrophobic medicine or the like in the hollow portion of the micelle, and it is preferable to enclose a hydrophilic medicine or the like in the hollow portion of the vesicle.

Such a structure has high degradation stability because the nucleic acid to be delivered is located inside the spherical structure. In addition, since the structure has the segment of the hydrophilic polymer in the outermost shell, the structure is excellent in blood retention. Then, the structure is enclosed in an endosome in a cell or the like as a target site and taken up into cytoplasm, then escapes from the endosome, and releases a nucleic acid to be delivered or a low molecular medicine as a drug in the cytoplasm or nucleus (see "BIOCHEMICAL EVALUATION" in the figure).

Examples of use of such a structure include carriers for drug delivery against various diseases. For example, in Examples described later, it is shown that the nucleic acid analog of the present invention is effective as a carrier for delivering, as a nucleic acid to be delivered, microRNA-143 that is an anti-oncomicroRNA to K-Ras of a cancer promoting gene and a network thereof.

The surface of the structure may be modified with various ligands. By modifying the surface of the structure with such a ligand, target directivity or the like can be imparted to the structure. Examples of the ligand include immunoglobulins, carbohydrates, peptides, proteins, aptamers, and the like. The main ligands and their application fields are as follows.
- Glucose: tumor study, drug delivery to brain capillary endothelial cells
- Mannose: efficient formation of large liposomes
- Galactose: study of galactose receptors in macrophages, study of targeted delivery of galactose to hepatocytes
- Sucrose: cancer treatment with doxorubicin
- Maltose: transport of doxorubicin in cancer treatment
- Lactose: studies of liposome size and stability
- Oligosaccharides: design of therapeutic inhibitors
- Lectin: pulmonary drug delivery
- Tomato lectin, wheat germ agglutinin: oral administration of insulin
- NCL-Aptamer: chemotherapy using cisplatin against wide range of cancers
- sgc8 Aptamer: for leukemia CEM-CCRF cells
- NX 1838: specific binding to VEGF of cancer cells
- Anti-CD44: selective targeting of cancer cells
- DAG-NX213: specificity to VEGF for promoting angiogenesis
- AS1411: cytotoxicity against breast cancer cells MCF-7
- Macugen: therapeutic agent for macular age-related macular degeneration
- BOCK: use for recognizing different binding sites of thrombin
- TASSET: use for recognizing different binding sites of target proteins
- xPSM-A9: use against prostate-specific membrane antigen expressed on prostate cancer cells
- IL-4Rα: suppression of tumor growth utilizing tumor microenvironment

### 3. Methods for producing nucleic acid analog and structure for nucleic acid delivery

### (1) Method for producing nucleic acid analog

Next, a method for producing a nucleic acid analog will be described. The nucleic acid analog can be produced by various methods, and examples thereof include a method of individually synthesizing a cationic artificial nucleic acid and a hydrophilic polymer and binding them. That is, the method for producing a nucleic acid analog includes a cationic nucleic acid synthesis step of synthesizing a cationic artificial nucleic acid, a hydrophilic polymer step of synthesizing a hydrophilic polymer, and a binding step of binding the cationic artificial nucleic acid and the hydrophilic polymer.

### (a) Two-step synthesis method

The cationic artificial nucleic acid can be synthesized by a two-step reaction (two-step synthesis method). As the two-step synthesis method, mainly three kinds can be mentioned. Hereinafter, the synthesis methods will be sequentially described.

### (a-1) Two-step synthesis method I

In this method, sulfurization (S-conversion) of a nucleic acid is performed by automatic nucleic acid synthesis, and a hydrophilic polymer phosphoramidite (for example, ethylene glycol phosphoramidite) is linked to the sulfurized nucleic acid to synthesize an oligo PS-hydrophilic polymer, and then a Br compound is further reacted to introduce a cationic group into the nucleic acid (TEG-PS oligo and HEG-PS oligo systems of Examples described later).

In the case of a nucleotide backbone, the cationic artificial nucleic acid can be synthesized by a method including the steps of: introducing a thiophosphate ester into a linking structure; and reacting a bromo compound having a cationic group with the thiophosphate ester to introduce a cationic group into the linking structure, as described in Examples described later.

### (a-1-2) Introduction of thiophosphate ester

The thiophosphate ester can be synthesized by a known phosphoramidite method. As an outline of the phosphoramidite method, a nucleoside or a nucleotide in which 5' is protected with a 4,4'-dimethoxytrityl (DMTr) group is supported on a solid phase (supporting step). Next, the DMTr group is deprotected with a deprotection reagent such as dichloroacetic acid (deprotection step), and coupled with a phosphoramidite nucleotide in the presence of an activating agent such as 4,5-dicyanoimidazole (coupling step). Thereafter, the phosphite ester is converted into a thiophosphate ester by a sulfurizing agent such as (N,N-dimethylaminomethylidene)amino]-3H-1,2,4-dithiazoline-3-thione (DDTT) (sulfurization step). Alternatively, the phosphite ester is converted into a phosphate diester by an oxidizing agent containing iodine, pyridine, or the like (oxidation step). By repeating this step, a synthetic nucleic acid containing a thiophosphate ester in the linking structure can be produced. By changing the type of the base of phosphoramidite, a synthetic nucleic acid having a desired sequence can be produced. Incidentally, it is also possible to introduce the thiophosphate ester only at a desired position of the linking structure constituting the nucleotide backbone by performing the sulfurization step instead of the oxidation step.

### (a-1-2)

Next, a bromo compound having an amine or an ammonium group is reacted with the obtained synthetic nucleic acid. Examples of the bromo compound include 3-bromo-1-propylamine hydrobromide, 2-bromo-N,N-diethylethylamine hydrobromide, (3-bromopropyl)trimethylammonium bromide, and the like The reaction between the synthetic nucleic acid and the bromo compound can be performed in a phosphate buffer or the like, and the reaction conditions can be appropriately set, but for example, the reaction can be performed at a pH within the range of 5 to 7, a reaction temperature of 30 to 60°C, and a reaction time of 10 to 50 hours.

### (a-2) Two-step synthesis method II

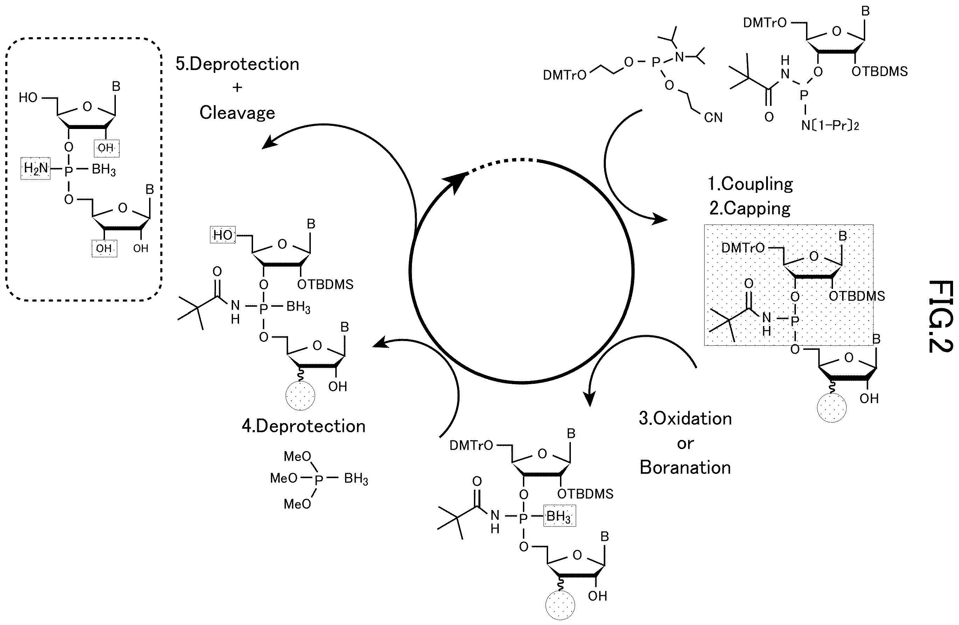

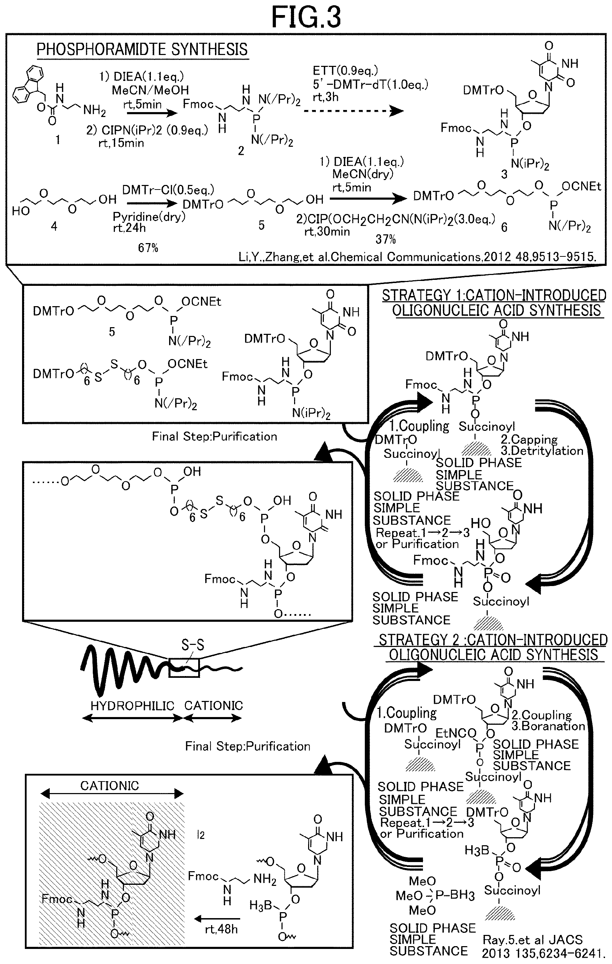

In this method, boranophosphation (B-conversion) of nucleic acid is performed by automatic nucleic acid synthesis, and a hydrophilic polymer phosphoramidite (for example, ethylene glycol phosphoramidite) is linked to the boranophosphated nucleic acid to synthesize an oligo B-hydrophilic polymer (Fig. 2). Thereafter, an amino group compound is further reacted by iodine oxidation to introduce a cationic group (strategy 2 in Fig. 3). In this method, it is also possible to introduce two cationic groups into one phosphate group (double cation introduction) by using a cationic phosphoramidite described later.

### (a-3) Two-step synthesis method III

In this method, a cationic artificial nucleic acid is synthesized by automatic nucleic acid synthesis, and then a hydrophilic portion is introduced by a click reaction (system of PEG-PMO in Examples described later). That is, it is a method in which a cationic artificial nucleic acid having a cationic group in the backbone is synthesized, and then a hydrophilic azide compound such as azide polyethylene glycol is linked thereto by a click reaction.

### (b) One-step synthesis method

The method described above is a two-step synthesis method in which a synthetic nucleic acid containing a thiophosphate ester is produced and a bromo compound is reacted therewith, but a cationic artificial nucleic acid can also be synthesized in one step. Fig. 2 illustrates a scheme of this method. The outline of this method is a one-step synthesis of a cation-hydrophilic polymer by automatic nucleic acid synthesis of cationic phosphoramidite and ethylene glycol phosphoramidite (strategy 1 in Figs. 2 and 3). This method is basically a scheme similar to the phosphoramidite method shown in the two-step synthesis method, but there are some differences.

First, a diisopropylamide phosphorous acid compound and a nucleotide monomer are reacted with the nucleotide supported on the solid phase ("1. Coupling" in the figure). Some hydroxyl groups are protected with a protecting group ("2. Capping"), and oxidized or borated with an oxidizing agent or a borating agent ("3. Oxidation or Boranation"). An amino group compound is reacted by iodine oxidation to introduce a cationic group.

Fig. 3 illustrates an example of a synthesis scheme of a nucleic acid analog. In this example, a scheme for synthesizing a nucleic acid analog having a nucleotide backbone as a cationic artificial nucleic acid and polyethylene glycol as a hydrophilic polymer is shown. In "PHOSPHORAMIDITE SYNTHESIS" in the figure, a phosphoramidite nucleotide is synthesized by a known method in the order of compounds 1 to 3. On the other hand, phosphoramidite polyethylene glycol is synthesized in the order of compounds 4 to 6.

Next, a nucleic acid analog is synthesized by the scheme shown in "STRATEGY 1: CATION-INTRODUCED OLIGONUCLEIC ACID SYNTHESIS" in the figure. Specifically, a phosphoramidite nucleotide and a phosphoramidite polyethylene glycol synthesized by the above scheme, and phosphoramidite disulfide having a disulfide bond and phosphoramidite in the molecule are used as raw materials. Then, a reaction such as protection with a protecting group and deprotection is performed to bond the hydrophilic polymer to the 5'-position of the cationic artificial nucleic acid. The cationic artificial nucleic acid may be synthesized in one step by solid phase synthesis as in the scheme shown in "STRATEGY 2: CATION-INTRODUCED OLIGONUCLEIC ACID SYNTHESIS" in the figure.

### (2) Method for producing structure for nucleic acid delivery

Next, a method for producing a structure for nucleic acid delivery will be described. The structure for nucleic acid delivery associates a nucleic acid to be delivered with a nucleic acid analog to form a complex (association step). When there is complementarity between a cationic group of the nucleic acid analog and a phosphate group of the nucleic acid to be delivered, both are bonded by annealing to form a double strand. The ratio between the nucleic acid analog and the nucleic acid to be delivered at the time of annealing is not particularly limited, but is preferably within the range of nucleic acid analog: nucleic acid to be delivered = 1: 1 to 1: 10. Annealing is performed by raising the temperature to a predetermined temperature and then lowering the temperature. As the annealing, the temperature is preferably raised to 80°C or more, and more preferably raised to 90°C or more. Also, the retention time of the raised temperature is preferably 5 minutes or more, and more preferably 10 minutes or more. Thereafter, the temperature is lowered to 50°C or less, preferably 30°C or less, and the temperature is maintained for 10 minutes or more, and preferably 30 minutes or more.

From the viewpoint of electrostatic interaction between the cationic group of the nucleic acid analog and the phosphate group of the nucleic acid to be delivered, this step is preferably performed under a pH condition lower than the pKa of the cationic group of the nucleic acid analog and higher than the pKa of the phosphate group of the nucleic acid to be delivered. That is, under such pH conditions, the cationic group is positively charged and the phosphate group is negatively charged, and these groups are electrostatically bonded. Such pH conditions depend on the pKa of the cationic group and the like, but the pH is preferably about 2 to 7, and more preferably about 3 to 6.

Next, a plurality of complexes are associated to form a higher order structure (associate forming step). In this step, the complexes are self-aggregated in an aqueous solvent such as water or an aqueous solution to form a structure such as a micelle or a vesicle. The concentration of the complex for forming a structure is about 25 to 2500 µM, and more preferably in the range of 100 to 1000 µM.

### 4. Nucleic acid delivery method

The nucleic acid delivery method of the present invention includes an administration step of administering the structure for nucleic acid delivery to incorporate the structure for nucleic acid delivery into a target site, and a releasing step of releasing the nucleic acid in the target site. In the administration step, the above-described structure in a state of being bound to the nucleic acid to be delivered is administered to a human, other mammals, or the like. A drug containing the structure is preferably administered into blood, but can be appropriately determined according to the disease to be treated, the type of hydrophilic polymer, and the like. The administered structure is taken up into cytoplasm via endosomes when the cell is the target site. In the releasing step, the nucleic acid to be delivered is released from the structure taken up into the cell as the target site. A structure in which the cationic group is highly pH-responsive such as quaternary ammonium is likely to collapse under an acidic environment in the endosome and release the nucleic acid to be delivered. As described above, it is also possible to enclose a low molecular medicine in the hollow portion of the structure and release the medicine in an evaluation site. By such a method, the nucleic acid to be delivered or the low molecular medicine can be delivered to the target site.

### Examples

Hereinbelow, the present invention is specifically described with reference to examples, but these examples do not limit the object of the present invention. In the following Examples, the expression "%" is based on mass (mass percent) unless otherwise specified.

### 1. Production of nucleic acid analog (artificial nucleic acid + hydrophilic polymer)

### (1-1) Oligonucleotide synthesis

Oligonucleotides (SEQ ID 1 to 4, 8, 12 to 15, 19 to 21, 25, 33, 37) were purchased from Gene Design Inc. (Osaka, Japan). As for the oligonucleotide to which the hydrophilic polymer was bonded, the phosphoramidite of ethylene glycol was linked by an automatic nucleic acid synthesizer at Gene Desing Inc., similarly to the nucleic acid sequence synthesis (the linking site was a phosphodiester bond).

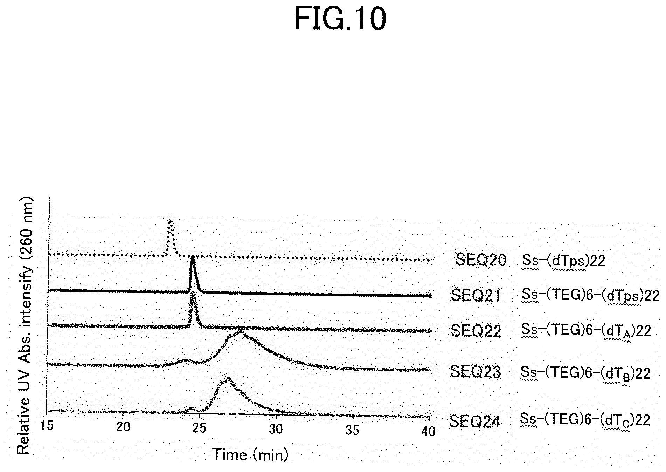

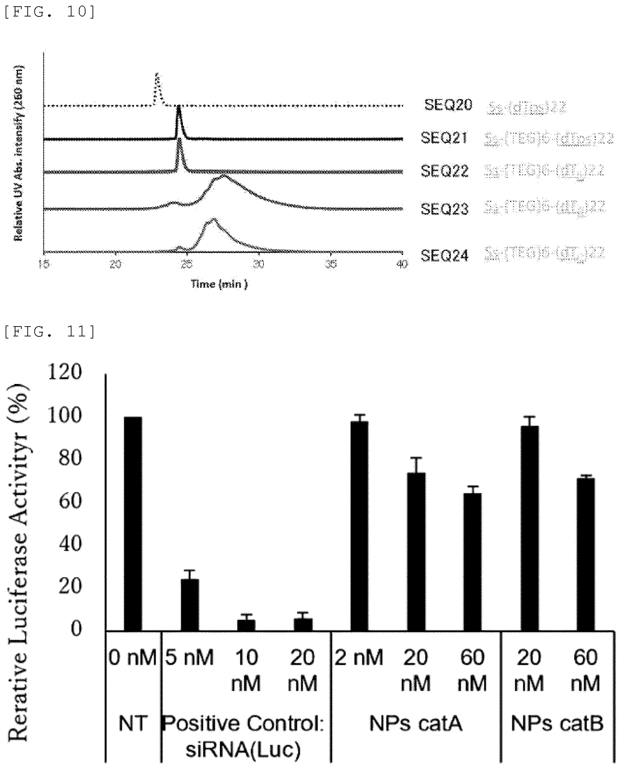

Oligonucleotides (SEQ ID 5 to 7, 9 to 11, 16 to 18, 22 to 24, 26 to 28) were each prepared by reacting at 45°C for 24 hours in a phosphate buffer (PBS: pH 6.5) using SEQ ID 4, 8, 15, 21, or 25 to which a hydrophilic polymer had been bonded in advance as a starting material, and a bromo compound (100 to 1000 eq). After the reaction, purification was performed by dialysis against distilled water for 5 to 7 days using Float-A-Lyzer G2, CE, 1 ml, 3.5 to 5 KDa (Repligen, Waltham, MA, US). After the purification, the mixture was concentrated by a freeze dryer to obtain a compound. The bromo compound 3-bromo-1 propylamine hydrobromide was purchased from Waco (Osaka, Japan). 2-Bromo-N,N-diethylethylamine hydrobromide and (3-bromopropyl)trimethylammonium bromide were purchased from Merck (Darmstadt, Germany). After the reaction, SEQ ID 22 to 24 were subjected to HPLC purification, desalting, and concentration to obtain products (Fig. 10). The elution time is shown in the following table.

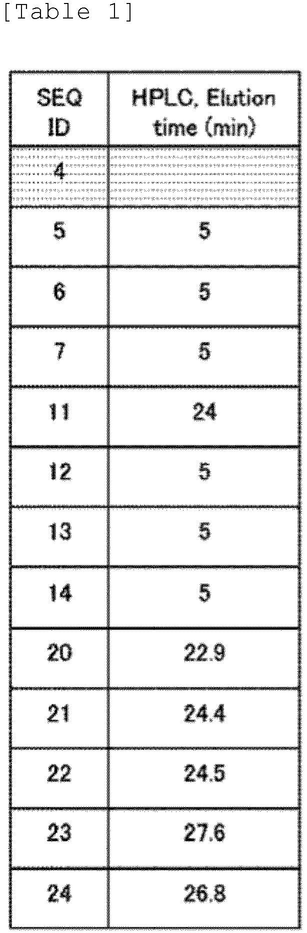

### (2) Morpholino nucleic acid

Morpholino nucleic acid (SEQ ID 29) was purchased from Gene Tools, LCC (Philomath, Pregon, US). Oligonucleotides (SEQ ID 30 to 32, 34 to 36, and 38 to 40) were each prepared by using SEQ ID 29, 24, or 28 as a starting material, and an azide polyethylene glycol compound (10 eq), with addition of copper(II) sulfate (10 eq) and sodium ascorbate (10 eq), and reacting the mixture in PBS (pH 7.4) at room temperature for 15 minutes to link hydrophilic polymers. After the reaction, the resultant was subjected to HPLC purification, desalting, and concentration to obtain a product. Poly(ethylene glycol)methyl ether azide as an azide compound, average Mn 1000 (10 K), 5000 (50 K), 10,000 (100 K), was purchased from Merck (Darmstadt, Germany).

### (3) Sequence list

The sequences of the synthesized oligonucleotides are shown in Tables 2 and 3. SEQ ID 1 to 2 indicate the antisense strands of microRNA-143 (AS-1,2), SEQ ID 3 to 11 indicate the sense strands of microRNA-143 (S-1 to 9), SEQ ID 12 to 13 indicate the antisense strands of siRNA against renilla luciferase, and SEQ ID 14 to 18 indicate the sense strands of siRNA against renilla luciferase (hereinafter, the antisense strand may be abbreviated as "AS", and the sense strand may be abbreviated as "S"). In this table, "N" (upper case) represents RNA, "dN" represents DNA, "N_{f}" represents 2'-FRNA, "Nₘ" represents 2'-OMeRNA, "X" represents -(OCH₂CH₂)₃O-, and "Y" represents -(OCH₂CH₂)₆O-. "*" means -P(O)OH-, "^" means - P(S)OH-, "¹" means -P(S-(CH₂)₃-NH₂)OH-, "²" means -P(S-(CH₂)₂-N(CH₂CH₃)₂)OH-, "³" means -P(-S(CH₂)₃-N(CH₃)₃)OH-, and "^{pmo}" means a morpholino nucleic acid (for some of these, refer to the following chemical formula. "^" indicates a structure in which two structures are conjugated.). "E" represents an ethynyl group, "L" represents a ligand compound (glucose, galactose, fucose, amino sugar), and "S" represents a disulfide.

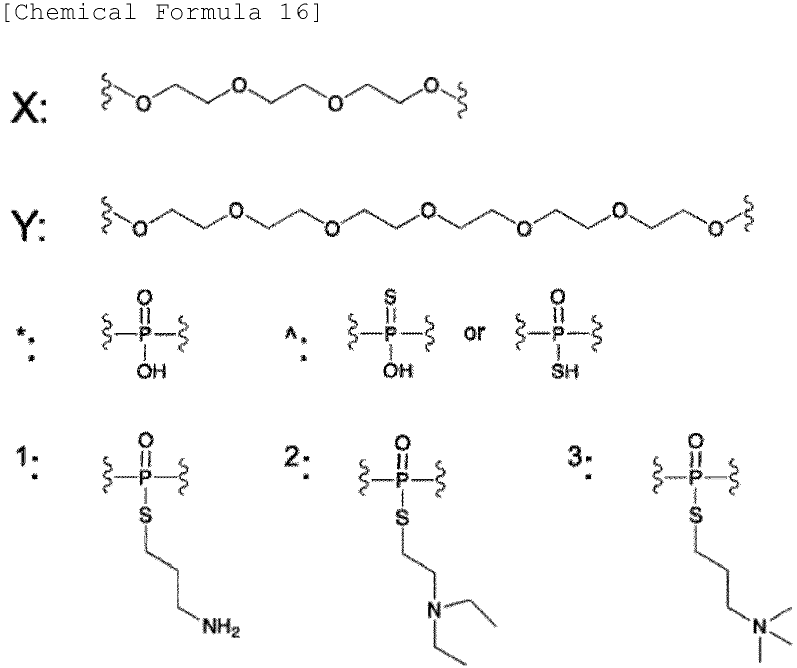

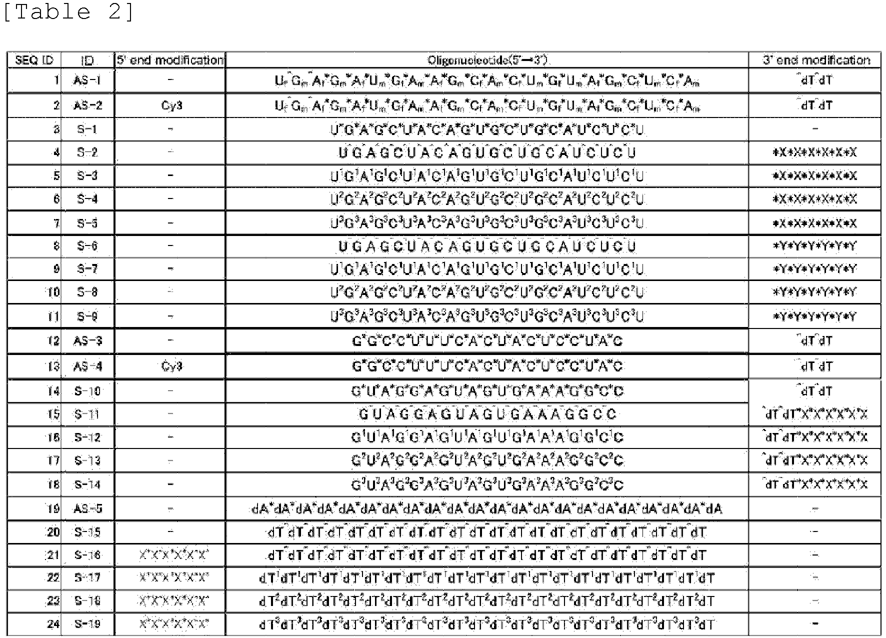

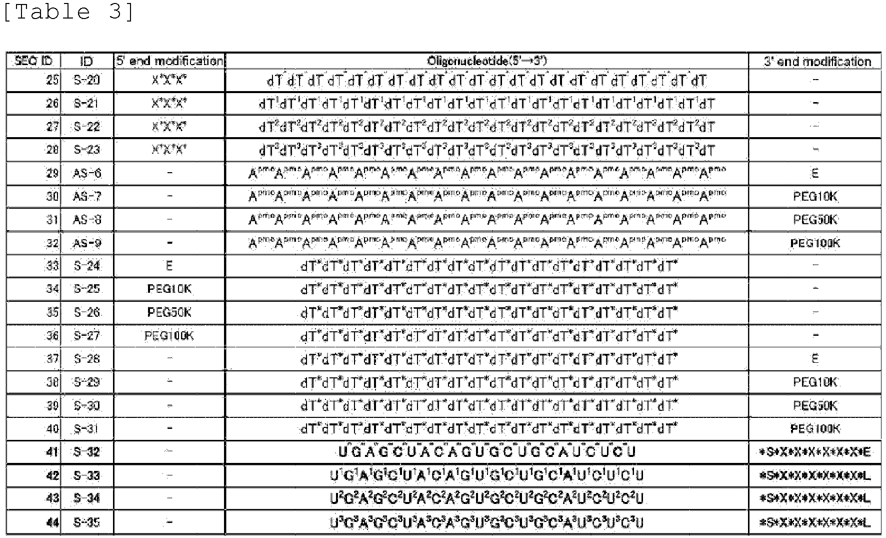

In the present specification, since a structure formed by association of a nucleic acid analog composed of a cationic artificial nucleic acid and a hydrophilic polymer with a nucleic acid to be delivered can reversibly take a state of a cationic polymer and a nucleic acid analog depending on conditions, the structure may be referred to as reversiblly ionic oligonucleotide-based nanoparticles and described as RION or RIO for convenience. When described as RION^{###} or RIO^{###}, the superscript# indicates the name of the nucleic acid to be delivered or the sequence of the nucleic acid. Furthermore, in the present specification, the amine may be described as Structure 1: primary amine (A), Structure 2: tertiary amine (B), Structure 3: quaternary ammonium (C), and the hydrophilic moiety may be described as Structure X: triethylene glycol (TEG), and Structure Y: hexaethylene glycol (HEG).
Example: RION¹⁴³ (TEG-B); Structure with triethylene glycol + microRNA-143 sequence + tertiary amine
Example: RIO¹⁴³(TEG-B); Structure with triethylene glycol + microRNA-143 sequence + tertiary amine

Hereinafter, the correspondence between the abbreviation (ID) and sequence number (SEQ ID) in the above table and Examples will be described.
"S-3" (SEQ ID 5) = Example 1,
"S-4" (SEQ ID 6) = Example 2,
"S-5" (SEQ ID 7) = Example 3,
"S-7" (SEQ ID 9) = Example 4,
"S-8" (SEQ ID 10 = Example 5,
"S-9" (SEQ ID 11) = Example 6,
"S-12" (SEQ ID 16) = Example 7,
"S-13" (SEQ ID 17) = Example 8,
"S-14" (SEQ ID 18) = Example 9,
"S-17" (SEQ ID 22) = Example 10,
"S-18" (SEQ ID 23) = Example 11,
"S-19" (SEQ ID 24) = Example 12,
"S-21" (SEQ ID 26) = Example 13,
"S-22" (SEQ ID 27) = Example 14,
"S-23" (SEQ ID 28) = Example 15,
"S-33" (SEQ ID 42) = Example 16,
"S-34" (SEQ ID 43) = Example 17,
"S-35" (SEQ ID 44) = Example 18

### 2. Method for forming structure and evaluation of physical properties

### (1) Annealing

Each nucleotide was dissolved in sterile water and then mixed in a weight ratio of AS: S = 1: 1 to 10. Thereafter, the mixtures were incubated at 98°C for 15 minutes, at 25°C for 50 minutes, and at 45°C for 50 minutes to obtain annealing mixtures (AN1 to 24 in the following table).

**[Table 4]**

| AN ID | ID | AN ID | ID | AN ID | ID | AN ID | ID |
|---|---|---|---|---|---|---|---|
| AN1 | AS-1 | AN10 | AS-3 | AN19 | AS-3 | AN2R | AS-7 |
| | S-1 | | S-10 | | S-5 | | S-25 |
| AN2 | AS-1 | AN11 | AS-3 | AN20 | AS-3 | AN29 | AS-7 |
| | S-2 | | S-11 | | S-6 | | S-28 |
| AN3 | AS-1 | AN12 | AS-3 | AN21 | AS-3 | | |
| | S-3 | | S-12 | | S-7 | | |
| AN4 | AS-1 | AN13 | AS-3 | AN22 | AS-5 | | |
| | S-4 | | 5-13 | | S-17 | | |
| AN5 | AS-1 | AN14 | AS-3 | AN23 | AS-5 | | |
| | S-5 | | S-14 | | S-18 | | |
| AN6 | AS-1 | AN15 | AS-3 | AN24 | AS-1 | | |
| | S-6 | | S-1 | | S-19 | | |
| AN7 | AS-1 | AN16 | AS-3 | AN25 | AS-3 | | |
| | S-7 | | S-2 | | S-17 | | |
| AN8 | AS-1 | AN17 | AS-3 | AN26 | AS-3 | | |
| | S-8 | | S-3 | | S-18 | | |
| AN9 | AS-1 | AN18 | AS-3 | AN27 | AS-3 | | |
| | S-9 | | S-4 | | S-19 | | |

### (2) Evaluation of physical properties of structure

Physical properties of the resulting annealing mixtures were evaluated using 20 wt% acrylamide gel electrophoresis (PAGE), transmission electron microscope (TEM), atomic force microscope (AFM), dynamic light scattering (DLS), circular dichroism (CD), and the like.

### (3) Native PAGE

The annealing mixture adjusted to 10 µM was applied 5 to 10 pmol to 7.5 or 20 wt% acrylamide gel and electrophoresed for 25 to 40 minutes. After electrophoresis, fluorescence of Cy3 was detected with Alliance Q9 (UVITEC, Cambridge, UK). After photographing, the nucleic acid was stained with SYBrGold for 5 minutes, and fluorescence of SYBR Gold Nucleic Acid Gel Stain (Thermo Fisher Scientific, Waltham, MA) was detected. As markers, 100 bp DNA Ladder (Takara Bio Inc., Shiga, Japan) and 20 bp DNA Ladder (Takara Bio Inc., Shiga, Japan) were applied to both ends of the sample.

### (4) TEM observation of structure

5 µL of a 10 µM annealing mixture was dropped onto a grid base, naturally dried, then stained with phosphotungstic acid, and observed with JEM-2010 (JEOL Ltd., Tokyo, Japan).

### (5) AFM observation of structure

5 µL of a 1.5 µM annealing mixture was dropped onto a mica substrate, naturally dried overnight, then vacuum-dried for 10 minutes, and observed with Nanocute (Hitachi High-Tech Corp., Tokyo, Japan).

### (6) Evaluation of particle size of structure by dynamic light scattering (DLS) measurement

DLS was measured at 37°C using DelsaMax PRO (Beckman Coulter, Brea, CA, USA). The concentration of the annealing mixture was 20 to 50 µM. The hydrodynamic radius (R_{H}) was calculated from autocorrelation function. The structural change by pH was adjusted by adjusting 1N HCl to pH 5. Serum resistance evaluation was performed by incubating in 50% FBS at 37°C and evaluating structural changes over time.

### (7) Chirality evaluation by circular dichriosm (CD)

CD was measured at 37°C using a quartz cell having an optical path length of 1 cm in a range of a wavelength of 240 to 320 nm and a temperature of 20 to 80°C using a circular dichroism spectropolarimeter (J-820KS; JASCO Corporation, Tokyo, Japan). The concentration of the annealing mixture was 1.5 µM.

### (8) Evaluation of formation of structure

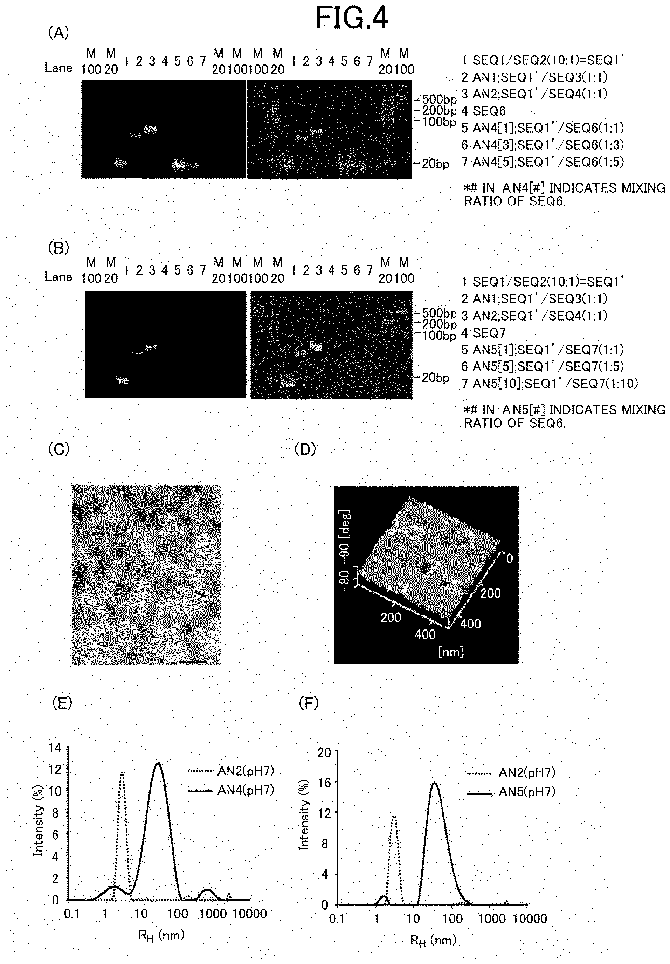

Fig. 4(A) illustrates electrophoretic results of the annealing mixture of AN4 (S-4 (Example 2) and AS-1), and Fig. 4(B) illustrates electrophoretic results of the annealing mixture of AN5 (S-5 (Example 3) and AS-1). In lanes 5 to 7 in the left diagram of (A) of the figure, a band of SEQ ID 1' derived from Cy3 (cyanine dye) was thinned. Here, SEQ ID 1' is obtained by mixing SEQ ID 1 and SEQ ID 2 at 9: 1, and Cy3 is derived from SEQ ID 2. This suggested the possibility of structure formation of SEQ ID 1' as the mixing ratio of SEQ ID 6 increased from 1 to 5. A similar tendency was observed in lanes 5 to 7 in the right figure after RNA staining after Cy3 detection. No band of SEQ ID 1' derived from Cy3 was detected in lanes 5 to 7 in the left diagram of (B) of the figure. This suggested that annealing mixing of SEQ ID 7 resulted in structure formation from the time point 1 regardless of the increase in mixing ratio of SEQ ID 7 from 1 to 10. In (C) of the figure, as a result of observing AN4 with TEM, a structure having a diameter of about 60 nm was observed. In (D) of the figure, as a result of observing AN5 with AFM, a structure having a diameter of about 75 nm was observed. From (E, F) of the figure, the hydrodynamic radii (R_{H}) of AN4 and AF5 were 35 nm and 45 nm, respectively. These results revealed that the annealing mixture forms a nanostructure. R_{H}s of other annealing mixtures are shown in the following table.

**[Table 5]**

| **AN ID** | ***R*_{H} (nm)** |
|---|---|
| **AN2** | **3.0** |
| **AN3** | **833** |
| **AN4** | **35** |
| **AN5** | **45** |
| **AN7** | **114** |
| **AN8** | **54** |
| **AN9** | **70** |
| **AN11** | **2.5** |
| **AN12** | **610** |
| **AN13** | **141** |
| **AN29(pH7)** | **2** |
| **AN29(pH4)** | **150** |

| | |
|---|---|
| * List of particle system of annealing mixtures (AN3 = RIO¹⁴³(TEG-A), AN4 = RIO¹⁴³(TEG-B), AN5 = RIO¹⁴³(TEG-C), AN7 = RIO¹⁴³(HEG-A), AN8 = RIO¹⁴³(HEG-B), AN9 = RIO¹⁴³(HEG-C)) | |

### (9) pH Response evaluation and serum resistance evaluation of structure

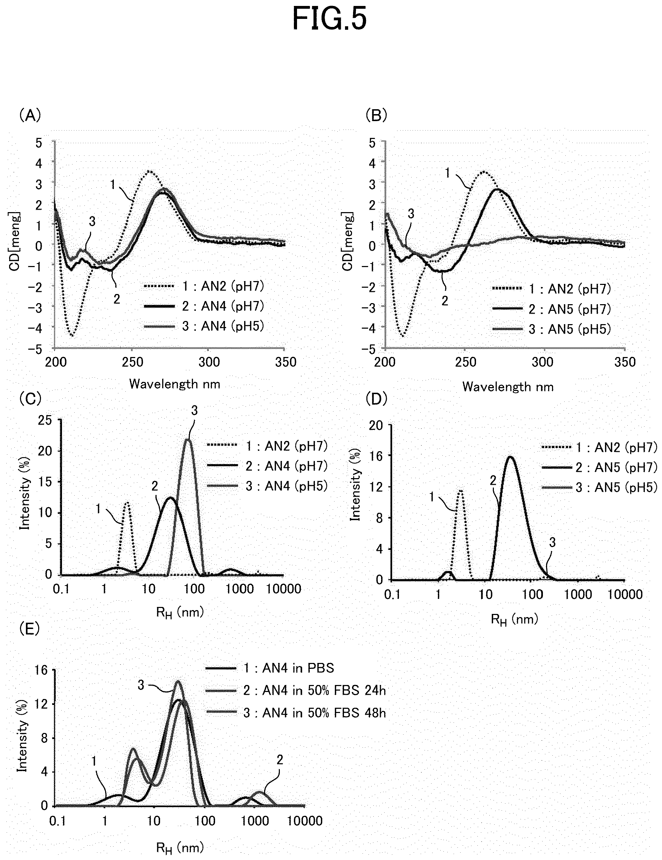

Fig. 5 illustrates results of evaluating structural change of the structure due to pH response and stability of the structure in serum. From (A, B) of the figure, under the condition of pH 7, in both AN4 and AN5, a positive peak at 260 nm, which is characteristic of A-type helical structure observed in AN2, was shifted to the longer wavelength side, and a severe negative dip at 210 nm was not observed. When the pH was changed to 5, no change was observed in AN4, whereas the intensity of the positive peak at 260 nm decreased in AN5. The results of measuring R_{H} at this time are shown in (C, D) of the figure. In AN4, R_{H} increased from 35 nm to 85 nm. On the other hand, in AN5, the strength of the autocorrelation function was remarkably reduced at pH 5, and R_{H} could not be calculated. From these results, in AN4, the cationic group is a tertiary amine, but it was revealed that chirality of the structure did not change greatly when the pH changed from 7 to 5, but R_{H} changed about twice. In AN5, the cationic group is quaternary ammonium, but it was suggested that chirality of the structure and R_{H} were greatly changed when the pH was changed from 7 to 5, and in particular, R_{H} could not be calculated, thus the structure collapsed with pH decrease. In (E) of the figure, serum resistance of AN4 over time was evaluated from R_{H}. No change was observed at 24 hours and 48 hours compared to R_{H} before incubation with serum, so that it was revealed that serum resistance was exhibited for a long time as 48 hours.

### (10) Cellular evaluation of nucleic acid delivery by structure

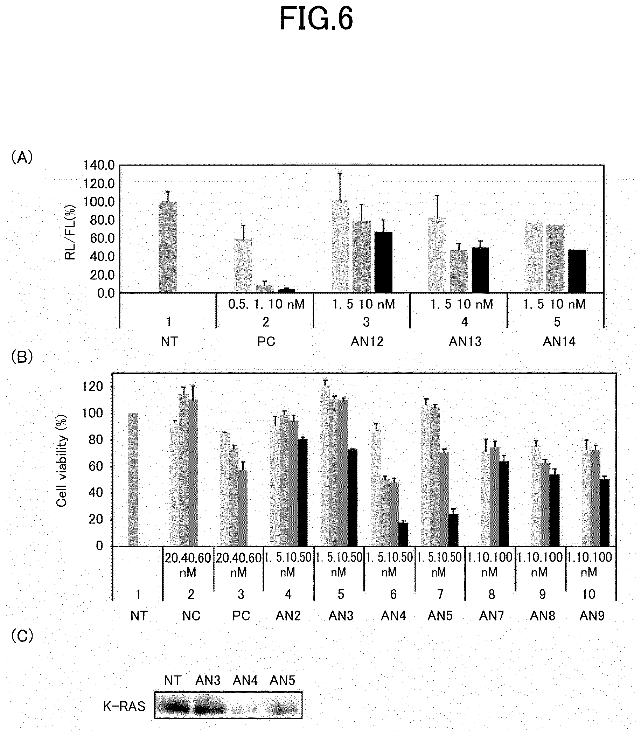

In Fig. 6, as the evaluation of nucleic acid delivery by structures, the results of inhibition evaluation of renilla luciferase (RL) activity using DLD-1 cell line ((A) of the figure) and evaluation of microRNA-143 delivery ((B, C) of the figure) are illustrated. In AN12 (S-12 (Example 7) and AS-3), AN13 (S-13 (Example 8) and AS-3), and AN14 (S-14 (Example 9) and AS-3), the tendency of inhibition of expression of RL was evaluated depending on the addition concentration, and the inhibitory activity by renilla luciferase sequence forming the structure was confirmed ((A) of the figure). In (B) of the figure, AN4 and AN5 significantly reduced the viability of DLD-1 in an added concentration-dependent manner. Proteins were extracted from DLD-1, and the presence of K-RAS, which is a target protein of microRNA-143, was evaluated ((C) of the figure). As a result, it was revealed that expression of K-RAS was suppressed in AN4 and AN5, and it was shown that AN4 and AN5 can deliver microRNA-143 in particular. The results for the other annealing mixtures are shown in the following table.

**[Table 6]**

| **AN ID** | **DLD-1 cell viability** | |
|---|---|---|
| | **% of NT** | **SD** |
| **AN1 (50 nM)** | **80.5** | **1.84** |
| **AN3 (50 nM)** | **72.9** | **0.31** |
| **AN4 (50 nM)** | **18.1** | **0.99** |
| **AN5 (50 nM)** | **24.5** | **3.9** |
| **AN7 (100 nM)** | **64.0** | **4.6** |
| **AN8 (100 nM)** | **54.4** | **3.9** |
| **AN9 (100 nM)** | **50.4** | **2.6** |

| | | |
|---|---|---|
| * List of DLD-1 viability by annealing mixture | | |

### (11) Confocal laser microscope observation

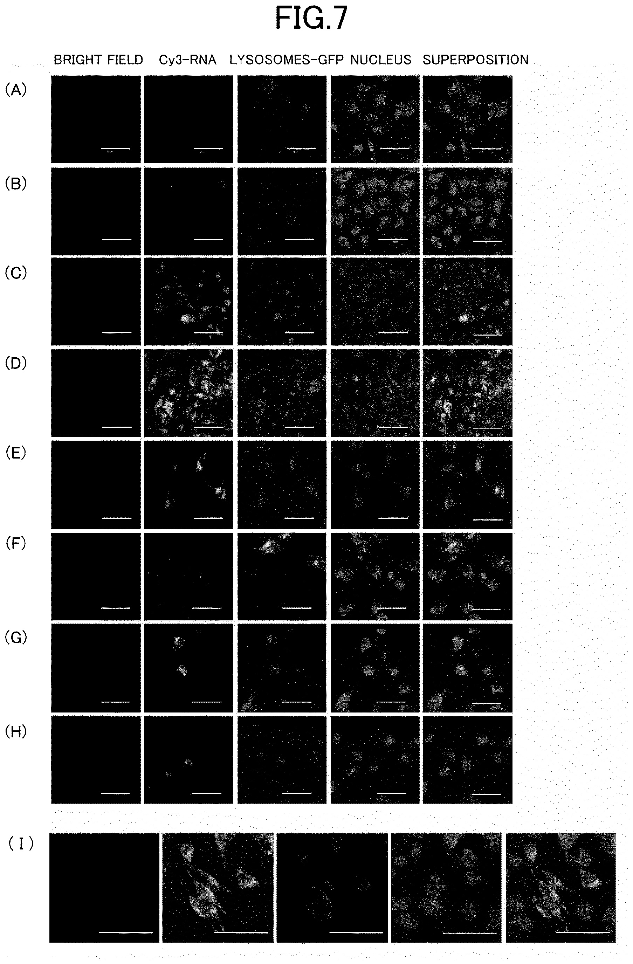

Uptake of each structure into DLD-1 cells was observed with confocal laser microscopy. In Fig. 7 (C, D, E, G), red fluorescence images of Cy3 derived from RNA were observed, and it was shown from superimposed images that Cy3 exists independently of green images of lysosomes. In particular, strong cellular uptake was observed for AN4 in (D) of the figure. (I) of the figure illustrates an image obtained by observing uptake of AN4 at 120-fold magnification. From the results, it was presumed that the structure was taken up into the cytoplasm without intersecting a cell nucleus or lysosome.

### 3. Evaluation of structure formation of morpholino nucleic acid (PMO)-PEG conjugate

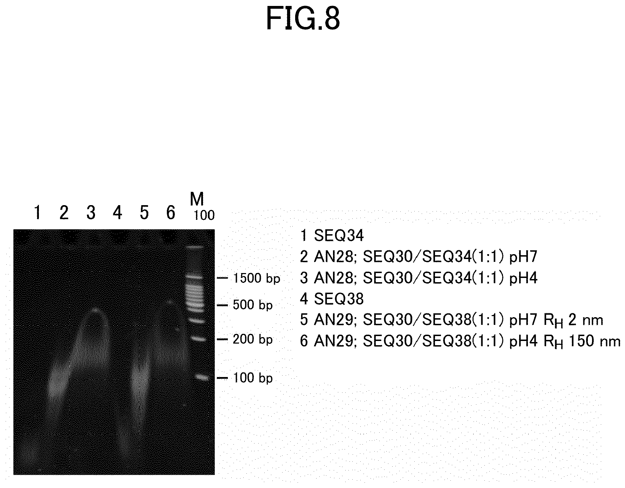

Fig. 8 illustrates results of electrophoresis at pH 7 and 4 of annealing mixtures (AN28 (S-25 and AS-7), AN29 (S-28 and AS-7)) using morpholino nucleic acid. After electrophoresis, the gel was RNA stained and photographed. The conditions for electrophoresis are 7.5 wt% acrylamide gel, Loading Volume: 10 pmol/well, Time: 40 min, Dye: Sybrgold, Detection: UV → Ebtrfillter. Lanes 1 and 4 are SEQ ID 34 and SEQ ID 38 before annealing. Lanes 1 and 4 are annealed with SEQ ID 30, respectively, and adjusted to pH 7 and pH 4. As a result, bands of AN28 and AN29 shifted to the polymer side when the pH changed from 7 to 4. Particle system measurement by DLS showed that AN29 (pH 7) was 2 nm (lane 5), and AN29 (pH 4) was 150 nm (lane 6). It was suggested that the annealing mixture using morpholino nucleic acid forms a structure by pH change.

### 4. Evaluation of non-complementary strand annealing mixture

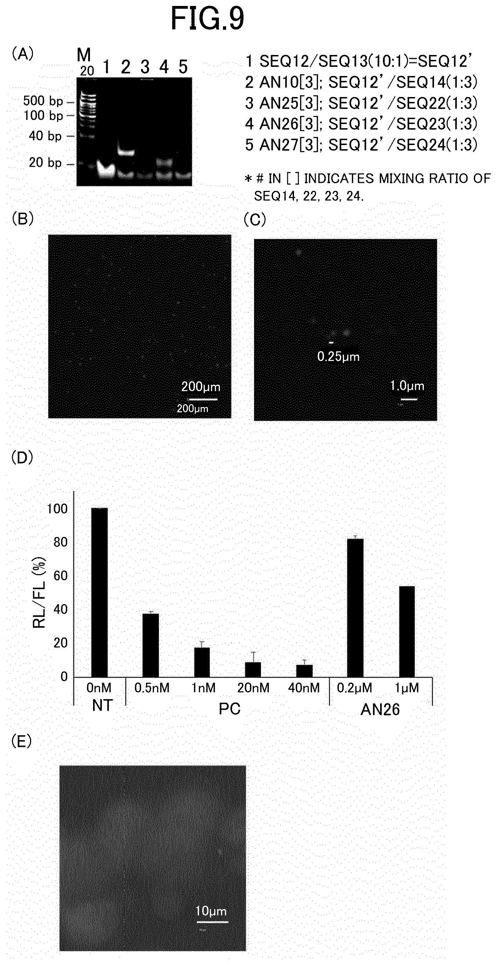

The electrophoretic results of non-complementary strand annealing mixtures (AN25 to 27) are shown in Fig. 9(A). In AN25 (S-17 (Example 10) and AS-3) in lane 3, a band of SEQ ID 12 (AS chain of luciferase) was thinned, and new fluorescence was observed in the well part. In AN26 (S-18 (Example 18) and AS-1) in lane 4, a band was observed at a new position. In AN27 (S-19 (Example 12) and AS-3) in lane 5, non-complementary strand interaction is not particularly observed. Subsequently, AN26 was observed with a confocal microscope ((B) and (C) of the figure). As a result, fluorescence of about 250 nm was observed, so that it was suggested that a structure was present. Using AN26, inhibition evaluation of renilla luciferase (RL) activity using MCF-7 cell line (human mammary gland cancer) was performed ((D) of the figure). Expression of RL was inhibited depending on the addition concentration of AN26. At this time, it is observed that AN26 is taken up into MCF-7 ((E) of the figure).

### 5. Evaluation of luciferase siRNA delivery using structure

### (1) Cell culture

Human colorectal cancer cell line DLD-1 (K-RAS mutant; G13D) and the human mammary gland cell line MCF-7 were purchased from the Japanese Collection Research Bioresouses Cell Bank. The DLD-1 cells were cultured using RPMI-1640 containing 10% inactivated FBS (Waco Inc., Osaka, Japan) under conditions of 5% CO₂, 37°C. The MCF7 cells were cultured using MEMα containing 10% inactivated FBS, non-essential amino acids, sodium pyruvate (Waco Inc., Osaka, Japan) under conditions of 5% CO₂, 37°C.

### (2) Luciferase inhibition evaluation

The DLD-1 and the MCF-7 were seeded in a 24-well plate at 5 × 10⁴ cell/well and cultured overnight. Thereafter, 100 ng of psiCheck2 (Promega Corporation, Madison, WI, USA) was added using Transfast (Waco Inc., Osaka, Japan). On the next day, the cells were collected, and then seeded in a 96-well plate at 5 × 10⁴ cell/well, and cultured overnight. Next, each annealing mixture was added so that the final concentration was 1-1000 nM, and cultured for 48 hours. Thereafter, the supernatant liquid was removed, and the plate was frozen at -200°C for 6 hours. Thereafter, the inhibitory activity of renilla luciferase was detected as a luminescence signal using Dual-Glo (registered trademark) Luciferase Assay System (Promega Corporation, Madison, WI, USA). The results are shown in Figs. 6(A), 9(D), and 11.

### 6. Evaluation of RNA medicine (miR-143) delivery using structure

### (1) Inhibitory effect of DLD-1 and MCF-7 proliferation by structure

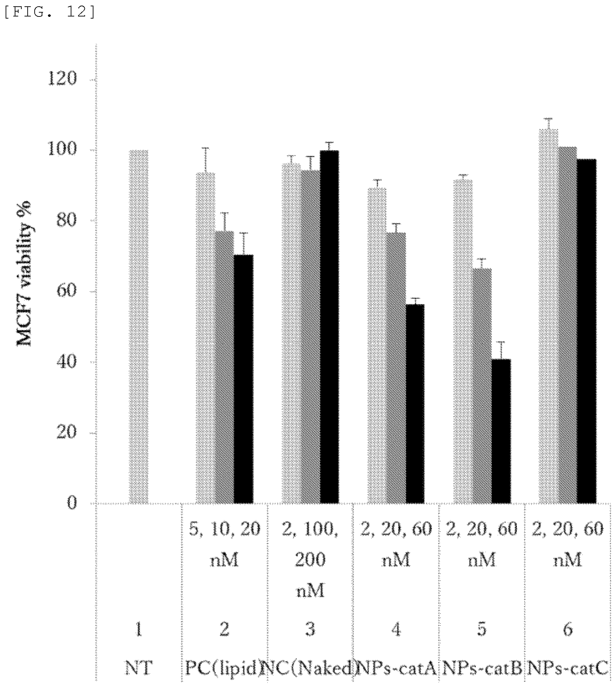

The DLD-1 and the MCF-7 were seeded in a 96-well plate at 1 × 10³ cell/well and cultured overnight. Thereafter, each annealing mixture was added so that the final concentration was 1-100 nM, and the mixture was cultured for 72 hours, then the remaining viable cells were detected as a light absorption signal using a cell proliferation/cytotoxicity assay kit (Cell counting WST-8; Waco Inc., Osaka, Japan). NT (non treatment) is a control in which no miRNA is introduced, and AN2 is a control (Naked) in which no lipofection reagent is used. The results are shown in Figs. 6(B) and 12.

### 7. Inhibitory mechanism of proliferation of DLD-1 and MCF-7 by structure

### (1) Western blot analysis

(a) Protein sample preparation: DLD-1 and MCF-7 were seeded in a 6-well plate at 1 × 10⁵ cell/well and cultured overnight. Thereafter, each annealing mixture was added so that the final concentration was 50 nM, and the mixture was cultured for 48 hours. Thereafter, the cells were peeled off with a scraper, and the cell suspension was collected in a 15 mL tube. After centrifugation to obtain cell pellets, protein extraction was performed. As a protein extract, 1% Protease inhibitor cocktail (Nakarai, Kyoto, Japan), Phosphatase inhibitor cocktail I, II (Merck Millipore, Burlington, MA, US), and III were mixed with protein lysis buffer (10 mM Tris-HCl, 0.1% SDS, 1% NP-40, 0.1% sodium deoxycholate, 150 mM NaCl, 1 mM EDTA) and used. The cell pellets were suspended in the protein extract and allowed to stand in ice for 20 minutes. Thereafter, centrifugation was performed at 15,000 rpm at 4°C for 20 minutes. The centrifuged supernatant was collected as a protein sample. The protein sample was adjusted to 20 µg/µL by mixing with SDS buffer (62.5 mM Tris-HCl, 2% SDS, 10% glycerol, 50 mM DTT, 0.01% bromophenol blue), boiled at 98°C for 5 minutes, and then allowed to stand on ice for 5 minutes.
(b) Electrophoresis, transfer, and detection

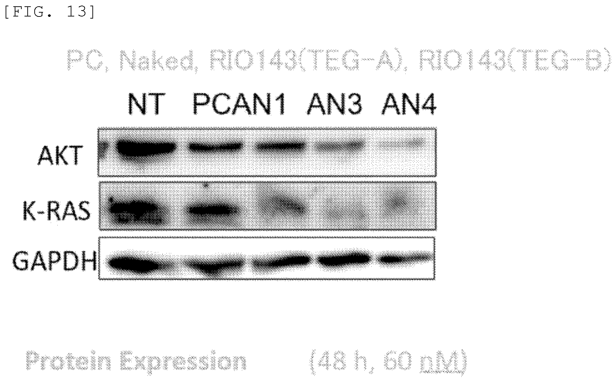

For electrophoresis, an easy separator (Wako Inc., Osaka, Japan) and Super Sep Ace (Wako Inc., Osaka, Japan) were used. After electrophoresis, the gel was immersed in Blotting buffer (25 mM Tris, 0.2 M glycine, 20% ethanol) for 5 minutes. A PVDF membrane (Perkin Elmer Life Sciences) was immersed in methanol for 10 minutes and immersed in ultrapure water for 10 minutes. Thereafter, the PVDF membrane was immersed in Blotting buffer for 5 minutes. From the anode side, a filter paper immersed in Blotting buffer, the PVDF membrane, the gel, and the filter paper were stacked in this order, and transferred at 15 V and 370 mA for 40 minutes. After transfer, the laminate was washed with a 50 mM Tris-HCl buffer (TBST) containing 0.1% Tween 20, and immersed in Brocking buffer (PVDF;) for 1 hour. The laminate was washed with TBST, and reacted overnight at 4°C in a primary antibody diluted with an antibody diluent (2% BSA, 0.01% sodium azide, TBST). After washing with TBST, the laminate was immersed in a secondary antibody and allowed to stand at room temperature for 1 hour. Thereafter, the sample was washed with TEST, allowed to emit light with Luminata Forte Western HRP Substrate (WBLUF0500; Merck Millipore, Burlington, MA, US), and then detected using GloMax Navigator (Promega Corporation, Madison, WI, USA). Anti-GAPDH (Cell Signaling Technology, Danvers, MA, US) was used as a control. Antibodies to K-RAS were purchased from Abcam (Cambridge, UK). The results are shown in Figs. 6(C) and 13.

### 8. Cellular uptake of structure

### (1) Observation of uptake of structure into DLD-1 and MCF-7

The DLD-1 and the MCF-7 were seeded in a 96-well plate at 5 × 10³ cell/well and cultured overnight. Thereafter, each annealing mixture was added so that the final concentration was 50 nM, and the mixture was cultured for 24 to 72 hours, then a fluorescence image was observed using a confocal laser microscope (FV3000; OLYMPUS, Tokyo, Japan). Nuclei were stained with Cellstain Hoechst 33342 (Wako Inc., Osaka, Japan), and lysosomes were stained with CellLight Lysosomes-GFP (Thermo Fisher Scientific, Waltham, MA).

### 9. Ligand artificial nucleic acid

### (1) Experimental data for oligonucleotide synthesis

Oligonucleotides (SEQ ID 41, 45) were purchased from Gene Design Inc. (Osaka, Japan). Oligonucleotides (SEQ ID 41 (E = Glu), SEQ ID 45 (E = Glu)) were each prepared by using SEQ ID 41 or 45 as a starting material, and an azide compound (10 eq), with addition of ascorbic acid and copper ions, and reacting the mixture in PBS (pH 7.4) at room temperature for 30 minutes (reaction reference: DOI: https://doi.org/10.1039/C0CC04979D). Here, "SEQ ID 41 (E = Glu)" means a ligand artificial nucleic acid to which glucose is bound as a ligand to a hydrophilic group of SEQ ID 41. As all solutions used in the reaction, a degassed solution was used. After the reaction, the reaction was confirmed by high performance liquid chromatography, and the resulting solution was fractionated. After the fractionation, the solution was desalted and concentrated by a freeze dryer. Thereafter, the mixture was reacted with bromo compound (100 to 1000 eq) in PBS at 45°C for 24 hours. After the reaction, dialysis was performed with distilled water using Float-A-Lyzer G2, CE, 1 ml, 3.5 to 5 KDa (Repligen, Waltham, MA, US) for 5 to 7 days. After dialysis, the solution was concentrated by a freeze dryer to obtain a compound (SEQ ID 43 (L = Glu), SEQ ID 47 (L = Glu)). The bromo compound (2-bromo-N,N-diethylethylamine hydrobromide) was purchased from Merck (Darmstadt, Germany). The sequences of the newly synthesized oligonucleotides are shown in Table 7, and the annealing mixtures are shown in Table 8.

**[Table 7]**

| SEQ ID | ID | 5' end modification | Oilgonucleolide(5'→3') | 3' end modification |
|---|---|---|---|---|
| 45 | S-36 | - | U^{^}G^{^}A^{^}G^{^}C^{^}U^{^}A^{^}C^{^}A^{^}G^{^}U^{^}G^{^}C^{^}U^{^}G^{^}C^{^}A^{^}U^{^}C^{^}U^{^}C^{^}U | *S*Y*Y*Y*Y*Y*E |
| 46 | S-37 | - | U¹G¹A¹G¹G¹U¹A¹C¹A¹G¹U¹G¹C¹U¹G¹C¹A¹U¹C¹U¹C¹U | *S*Y*Y*Y*Y*Y*Y*L |
| 47 | S-38 | - | U²G²A²G²C²U²A²C²A²G²U²G²C²U²G²C²A²U²C²U²C²U | *S*Y*Y*Y*Y*Y*Y*L |
| 48 | S-39 | - | U³G³A³G³C³U³A³G³A³G³U³G³C³U³G³C³A³U³C³U³C³U | *S*Y*Y*Y*Y*Y*Y*L |

**[Table 8]**

| AN ID | ID |
|---|---|
| AN30 | AS-1 |
| | S-34(L=Glu) |
| AN31 | AS-1 |
| | S-38(L=Glu) |

### (2) Antitumor effect: in vivo experiment

Mice subjected to the experiment were bred in accordance with the animal experiment guidelines of Hiroshima University. Tumor-bearing mice were prepared by transplanting colorectal cancer cells (DLD-1 cells, 8.0 × 10⁵ cells) suspended in Hank's solution subcutaneously into the back of mice. A sample was administered via the tail vein of the mice once every three days (four times in total) after solid cancer engraftment (7 days after transplantation). The samples administered are physiological saline, AN1, and AN4 (volume 100 µL, nucleic acid (guide chain) 375 ug/kg). The major axis (L mm) and minor axis (S mm) of the tumor of the mice were measured once every three days with a caliper, and tumor deposition (V mm³) was calculated from volume formula: V = (L × S²) × 0.5. Antitumor effect was evaluated from volume change of the tumor of the tumor-bearing mice.

### (3) Antitumor effect

Tumor-bearing mice were prepared by transplanting colorectal cancer cells (DLD-1 cells, 8.0 × 10⁵ cells) suspended in Hank's solution subcutaneously into the back of mice. After solid cancer engraftment (7 days after transplantation), physiological saline, AN1, and AN4 were administered via the tail vein (volume 100 µL, nucleic acid (guide chain) 375 µg/kg) once every three days (four times in total). The major axis (L mm) and minor axis (S mm) of the tumor of the mice were measured once every three days with a caliper, and tumor deposition (V mm³) was calculated from volume formula: V = (L × S²) × 0.5. Antitumor effect was evaluated from volume change of the tumor of the tumor-bearing mice.

### (4) Kinetic evaluation

AN1 and AN4 were administered via the tail vein to tumor-bearing mice (volume 100 µL, nucleic acid (guide chain) 375 gg/kg), blood was collected from the tail vein over time (1, 2, 4, 8, 24 hours), the fluorescence intensity of Cy3 was measured, and the biological half-life of the administered nucleic acid in blood was determined to be about 2.4 hours. In addition, when each organ was recovered 24 hours later and the fluorescence intensity of Cy3 was measured, no fluorescence of Cy3 was observed in the liver, and accumulation of the administered nucleic acid in the liver was not detected, but it was confirmed that fluorescence of Cy3 was observed and accumulated in tumor cells. According to a previous study in which RNAs were administered by LNP (Chen, S., et al, Journal of Controlled Release, 235, 236-244 (2016)), the half-life of the administered nucleic acid in blood was 0.25 hours, and the accumulation rate in the liver was 91.4%.

### (5) In vivo antitumor effect of AN4 (RIO¹⁴³(TEG-B))

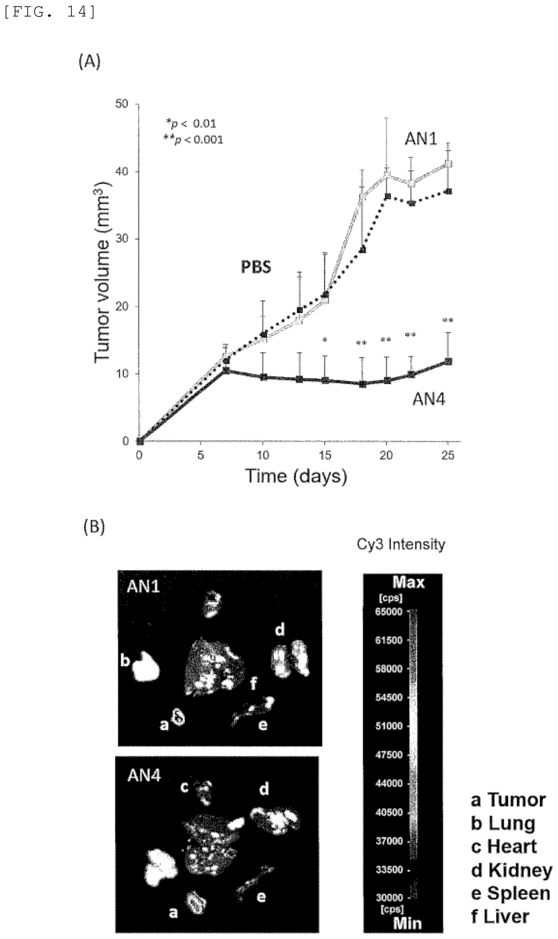

AN4 with demonstrated in vitro efficacy was developed in vivo to evaluate antitumor effects and tissue kinetics in colorectal cancer-bearing mice. The results are shown in Fig. 14. From 7 days after transplantation of the cancer cells, the sample was administered via the tail vein of the mice, and the volume change of the tumor was observed. In the group to which AN4 was administered, an increase in tumor volume was suppressed as compared with the group to which physiological saline or AN1 (Naked¹⁴³) was administered, and induction of an antitumor effect was observed. In kinetic evaluation, a fluorescent-labeled AN4 was administered via the tail vein of the tumor-bearing model mice, and 24 hours later, each organ was collected, and the fluorescence intensity was measured to measure uptake into the organ. As a result, AN4 was highly taken up by the tumor and accumulated only slightly in the liver. This result is expected to be developed as another transport technique for avoiding accumulation in the liver, which is a problem of lipid nanoparticles (LNP) widely used as carriers of nucleic acids.

### (6) Intracellular uptake of ligand RION^{miR-143}

DDS in systemic administration has "passive targeting" and "active targeting". In passive targeting, utilization of Enhanced Permeability and Retention effect (EPR effect), which is a characteristic of vascular endothelium around a tumor, is known. Active targeting is expected to deliver nucleic acids efficiently to cancer at lower dosages by delivering specifically only to target cells and tissues. Therefore, a ligand introduced structure (AN30) was examined as active targeting.

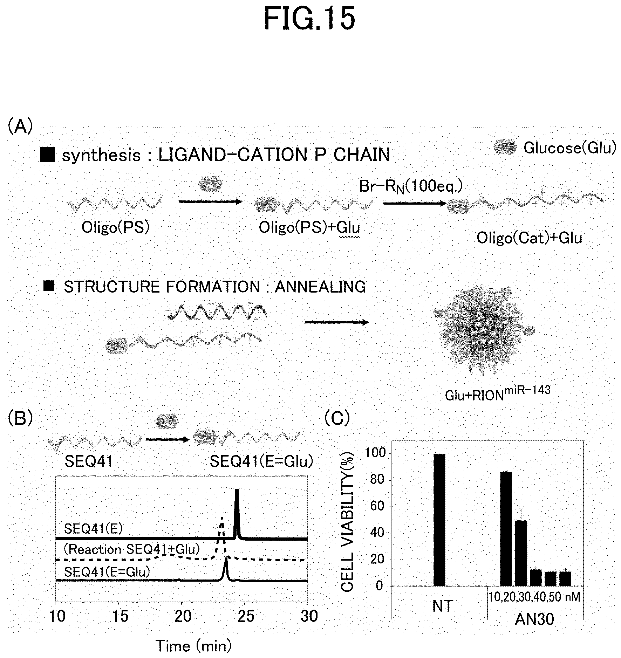

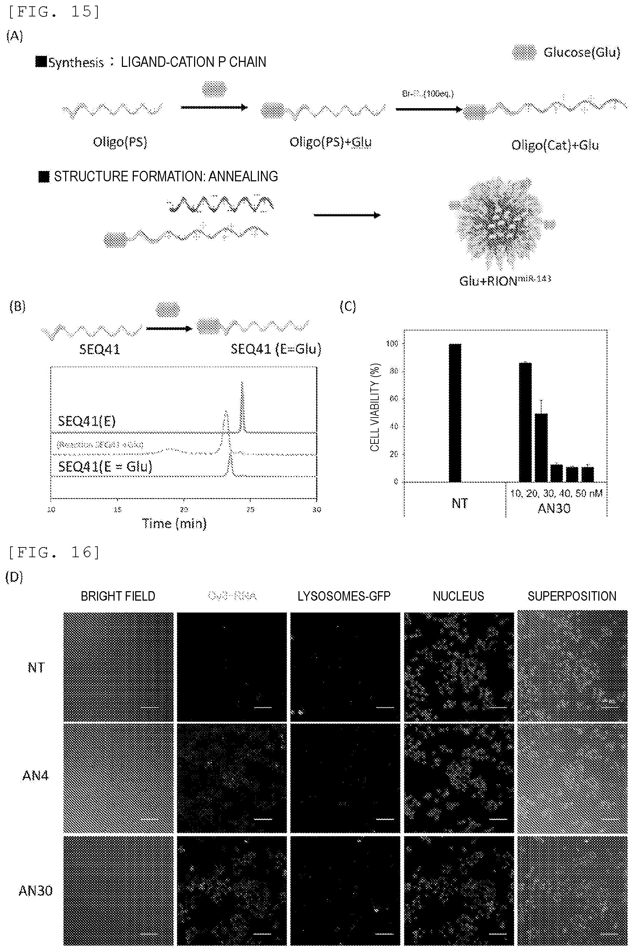

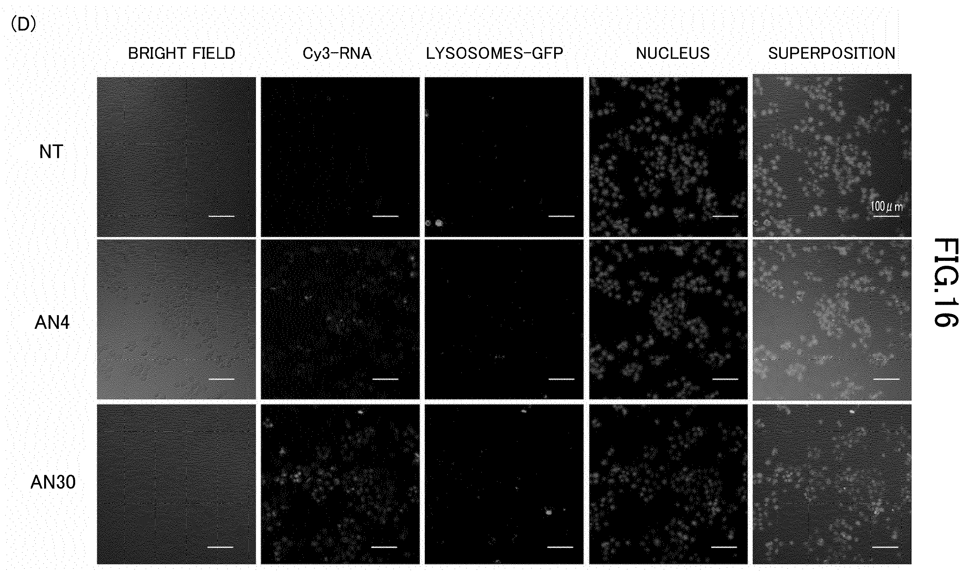

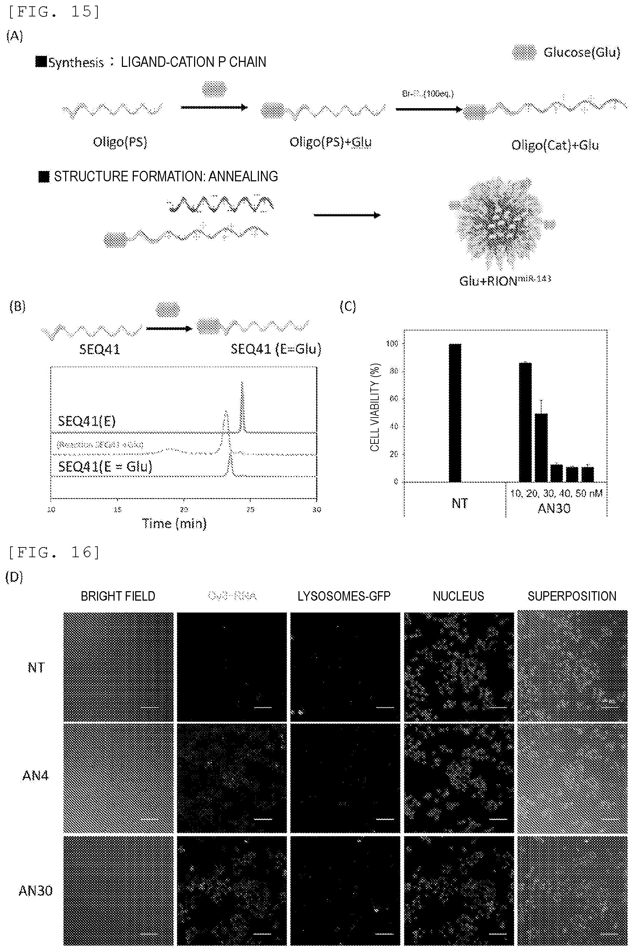

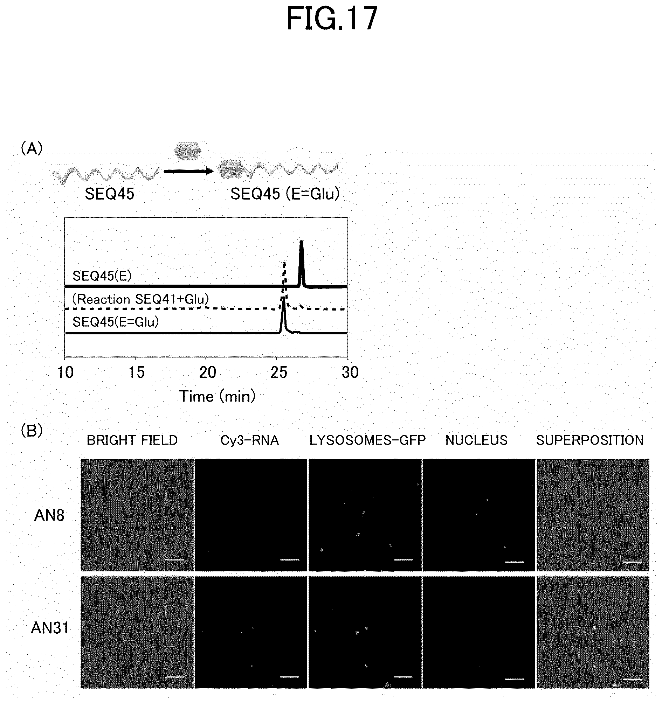

The results are shown in Figs. 15, 16, and 17. Fig. 15(a) is a diagram showing a scheme in which glucose (Glu) as a ligand is bound to a hydrophilic group of an artificial nucleic acid to form a structure together with RNA to be delivered. Fig. 15(B) and Fig. 17(A) are diagrams before and after a reaction in which glucose as a ligand is bound to an RNA strand. Fig. 16(D) and Fig. 17(B) are views showing that the delivered RNA is taken up into a cell. As a result of observing cellular uptake of AN30 with a fluorescence microscope, red fluorescence derived from RNA was observed after 6 hours from the addition, so that it was found that AN30 was taken up into the cell. In the observation of cellular uptake of AN31, a red tendency derived from RNA was observed after 24 hours from the addition, so that it was found that AN31 was taken up into the cell. In the evaluation of the cytostatic inhibitory effect in Fig. 15(C), AN30 suppressed survival of DLD-1 cells in an added concentration-dependent manner.

## Claims

1. A nucleic acid analog comprising a cationic artificial nucleic acid and a hydrophilic polymer bonded to the cationic artificial nucleic acid,
the cationic artificial nucleic acid having a backbone structure including a constituent unit in which a base is bonded to a ring structure selected from ribose and deoxyribose, and a linking structure linking the two constituent units,
the linking structure, in a cationic state, having a cationic group selected from the group consisting of the following formulae (C1) to (C7), and the cationic artificial nucleic acid being capable of associating by electrostatic interaction between a phosphate group of another nucleotide and the cationic group, 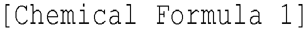 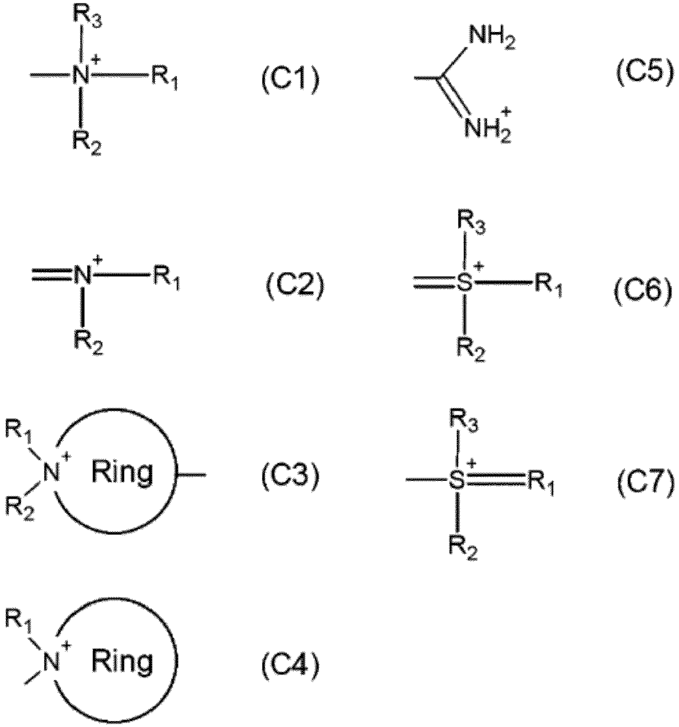
wherein R₁ to R₃ represent hydrogen or an alkyl group having 1 to 10 carbon atoms, R₁ to R₃ may be the same or different, and Ring is a cyclic compound composed of 4 to 8 carbon atoms, and may be a heterocyclic ring in which one or more of the carbon atoms are substituted with a heteroatom selected from nitrogen, oxygen, and sulfur.

2. The nucleic acid analog according to claim 1, wherein the cationic group has a pKa in the range of 6 to 9.

3. The nucleic acid analog according to claim 1, wherein the linking structure, in a cationic state, has at least a structure selected from the following formulae (L1) to (L4): 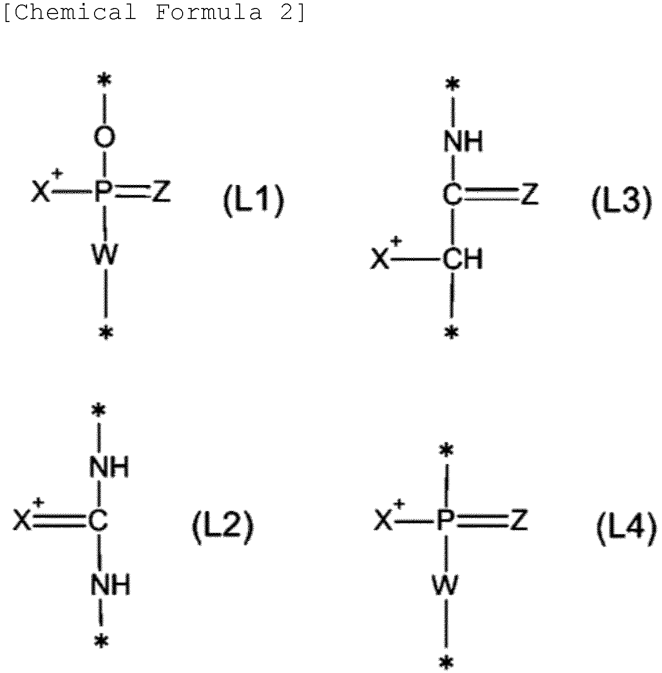 wherein X⁺ is a functional group containing the cationic group, Z represents O or S, W represents -O- or -NR₄-, and R₄ represents hydrogen or an alkyl group having 1 to 10 carbon atoms, and * means a bond with the constituent unit adjacent to each other.

4. The nucleic acid analog according to claim 1, wherein the cationic artificial nucleic acid has a nucleotide backbone represented by the following formula (N1) : 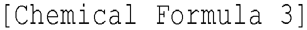 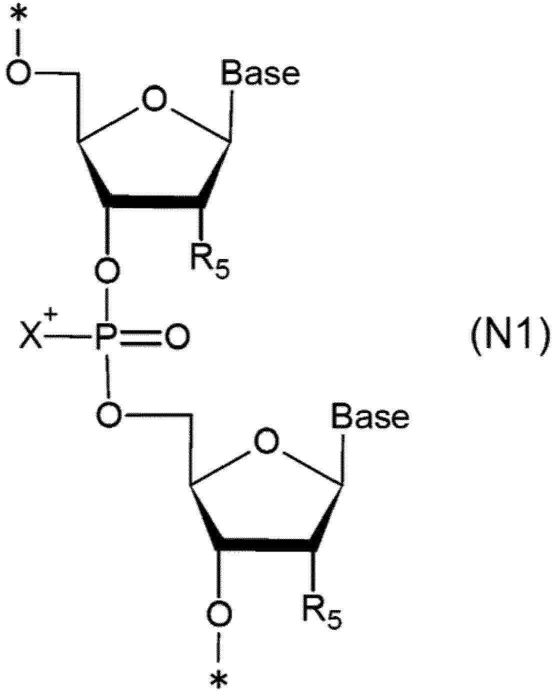 wherein X⁺ is a functional group containing the cationic group, Base represents a base, and R₅ represents H or OH, * means a bond with phosphoric acid of an adjacent nucleotide backbone, and at least one of " is bonded to the hydrophilic polymer at the 5' end or 3' end, and when not bonded to the hydrophilic polymer, * is hydrogen.

5. The nucleic acid analog according to claim 4, wherein the X⁺, in a cationic state, is an ammonium cation represented by the following formula (F1): 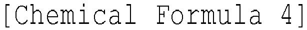 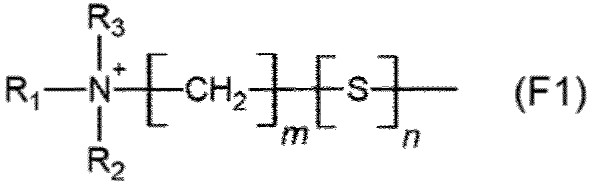 wherein R₁ to R₃ represent hydrogen or an alkyl group having 1 to 10 carbon atoms, and may be the same or different from each other, m represents an integer of 0 to 10, and n represents an integer of 0 or 1.

6. The nucleic acid analog according to claim 1, wherein the hydrophilic polymer is selected from polyethylene glycol, polyvinyl alcohol, polyglutamic acid, polyvinylpyrrolidone, polyacrylamide, polyethyleneimine, polyalkyl acrylate, polyoxazoline, polyacrylamide, poly(carboxybetaine methacrylate), poly(sulfobetaine methacrylate), poly(2-methacryloyloxyethyl phosphocholine), hyaluronic acid, chitosan, dextran, and derivatives thereof.

7. The nucleic acid analog according to claim 7, wherein the hydrophilic polymer has a polyethylene glycol backbone represented by the following formula (A1): 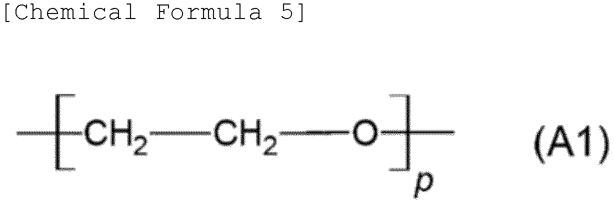 wherein p represents an integer of 1 to 20.

8. The nucleic acid analog according to claim 1, which is a carrier for delivering a nucleic acid to be delivered to a target site.

9. A structure for nucleic acid delivery comprising the nucleic acid analog according to claim 8 and a nucleic acid to be delivered, for delivering the nucleic acid to a target site, the structure for nucleic acid delivery having an association structure in which the cationic artificial nucleic acid and the nucleic acid are associated by electrostatic interaction.

10. The structure for nucleic acid delivery according to claim 9, wherein the structure for nucleic acid delivery is a nanoscale structure in which a plurality of the structures for nucleic acid delivery are associated with each other.

11. The structure for nucleic acid delivery according to claim 10, which is a vesicle or a micelle in which the association structure is located on an inner side and the hydrophilic polymer is located on an outer side.

12. The structure for nucleic acid delivery according to claim 11, which is modified with a ligand.

13. The structure for nucleic acid delivery according to claim 11, further comprising a medicine.

14. The structure for nucleic acid delivery according to claim 13, wherein the structure for nucleic acid delivery is a spherical structure having a hollow portion in a central portion, and
a medicine is enclosed in the hollow portion.

15. A method for producing the nucleic acid analog according to claim 1, comprising: a cationic nucleic acid synthesis step of synthesizing the cationic artificial nucleic acid; and a binding step of binding the cationic artificial nucleic acid and the hydrophilic polymer, and
the cationic nucleic acid synthesis step comprising: a step of introducing a thiophosphate ester into the linking structure; and a step of reacting the thiophosphate ester with a bromo compound.

16. A method for producing the structure for nucleic acid delivery according to claim 9, comprising:
an association step of associating the nucleic acid analog and the nucleic acid by electrostatic interaction under a pH condition lower than pKa of the cationic group of the nucleic acid analog and higher than pKa of phosphoric acid of the nucleic acid to form an association structure.

17. The method for producing the structure for nucleic acid delivery according to claim 16, further comprising: an associate forming step of associating a plurality of the structures for nucleic acid delivery to form, in an aqueous solvent, a vesicle or a micelle in which the association structure is located on an inner side and the hydrophilic polymer is located on an outer side.

18. A nucleic acid delivery method for delivering the nucleic acid to a target site using the structure for nucleic acid delivery according to claim 9, the nucleic acid delivery method comprising:
an administration step of administering the structure for nucleic acid delivery to incorporate the structure for nucleic acid delivery into the target site; and
a releasing step of releasing the nucleic acid in the target site.
